(19) Europäisches Patentamt / European Patent Office / Office européen des brevets

(11) **EP 4 582 993 B1**

(12) **EUROPEAN PATENT SPECIFICATION**

(45) Date of publication and mention
of the grant of the patent:
**24.12.2025 Bulletin 2025/52**

(21) Application number: **24305008.5**

(22) Date of filing: **03.01.2024**

(51) International Patent Classification (IPC):
**G06F 30/20** (2020.01)     **G06F 111/06** (2020.01)

(52) Cooperative Patent Classification (CPC):
**G06F 30/20;** G06F 2111/06

(54) **OPTIMIZATION OF ONE OR MORE ANISOTROPIC MATERIAL PROPERTIES OF A COMPOSITE PART**

OPTIMIERUNG EINER ODER MEHRERER ANISOTROPER MATERIALEIGENSCHAFTEN EINES VERBUNDTEILS

OPTIMISATION D'UNE OU DE PLUSIEURS PROPRIÉTÉS DE MATÉRIAU ANISOTROPE D'UNE PIÈCE COMPOSITE

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(43) Date of publication of application:
**09.07.2025 Bulletin 2025/28**

(73) Proprietor: **Dassault Systèmes**
**78140 Vélizy-Villacoublay (FR)**

(72) Inventors:
• **SCHMIDT, Martin-Pierre**
**Vélizy-Villacoublay (FR)**
• **PEDERSEN, Claus Bech Wittendorf**
**Hamburg (DE)**

(74) Representative: **Bandpay & Greuter**
**11 rue Christophe Colomb**
**75008 Paris (FR)**

(56) References cited:
**US-A1- 2023 114 354     US-A1- 2023 274 048**

**Description**

**TECHNICAL FIELD**

The disclosure relates to the field of computer programs and systems, and more specifically to a method, system and program for optimization of one or more anisotropic material properties of a composite part.

**BACKGROUND**

A number of solutions, hardware and software, are offered on the market for the design, the engineering and the manufacturing of objects. CAD is an acronym for Computer-Aided Design, e.g. it relates to software solutions for designing an object. CAE is an acronym for Computer-Aided Engineering, e.g. it relates to software solutions for analyzing and simulating the physical behavior of a future product. CAM is an acronym for Computer-Aided Manufacturing, e.g. it relates to software solutions for defining product manufacturing processes and resources. In such computer-aided design solutions, the graphical user interface plays an important role as regards the efficiency of the technique. These techniques may be embedded within Product Lifecycle Management (PLM) solutions. PLM refers to an engineering strategy that helps companies to share product data, apply common processes, and leverage corporate knowledge for the development of products from conception to the end of their life, across the concept of extended enterprise. The PLM solutions provided by Dassault Systèmes (under the trademarks CATIA, SIMULIA, DELMIA and ENOVIA) provide an Engineering Hub, which organizes product engineering knowledge, a Manufacturing Hub, which manages manufacturing engineering knowledge, and an Enterprise Hub which enables enterprise integrations and connections into both the Engineering and Manufacturing Hubs. All together the solutions deliver common models linking products, processes, resources to enable dynamic, knowledge-based product creation and decision support that drives optimized product definition, manufacturing preparation, production and service.

Within this context, CAD solutions exist to design composite parts. Such parts often feature anisotropic material properties and/or are formed in anisotropic materials.

Anisotropic materials can be found everywhere from the simplest wooden table or plywood wall to the most advanced ultralight carbon-fiber composite aerospace fuselage. An anisotropic material is a material having one or more physical properties (*e.g.* stiffness, thermal conductivity) that are directionally-depending. Among those materials are orthotropic materials. An orthotropic material is an anisotropic material where there are two mutually perpendicular planes of symmetry in the material properties.

FIG. 1 shows an example of a composite part which is a table formed by anisotropic wood beams having fibers aligned along the beam length direction, and isotropic plastic hubs with isotropic void space as shown in the figure.

FIG. 2 shows another example of a composite part which is an aircraft wind turbine blade (a cross section view of the blade being shown on FIG. 2), formed by anisotropic conformal composite laminate around an isotropic foam core.

Before fabrication, these objects are typically designed in CAD software. This allows defining a parameterized representation of the 3D part including various properties about the location, orientation and properties of the anisotropic material (see Webinar Composites in 3DEXPERIENCE CATIA: https://youtu.be/omaMz bnTEE).

For example, in the case of the wind turbine blade or aircraft fuselage, this digital representation can then be tested/simulated/optimized using a CAE software to validate or optimize its structural performance. Frequently, the optimization of such structures is carried-out as multiple sequential optimizations each acting on a subset of homogeneous variables and where each optimization step requires a different representation of the object. For example, one could run an initial topology optimization, as discussed in Bendsøe MP, Sigmund O (2003) Topology optimization theory, methods, and applications. Springer, Berlin, using a density field having isotropic material properties for obtaining a rough design idea and estimate. Then the topology optimized design can be post-processed, as discussed in Olhoff N, Bendsøe MP, Rasmussen J. On CAD-integrated structural topology and design optimization. Comput Methods Appl Mech Eng. 1991;89(1), and following fine-tuned using a surface-based shape optimization, as discussed in Olhoff N, Bendsøe MP, Rasmussen J. On CAD-integrated structural topology and design optimization. Comput Methods Appl Mech Eng. 1991;89(1), Fallahpour M, Harbrecht H. Shape optimization for composite materials in linear elasticity. Optimization and Engineering. 2022; 24(7), and Olhoff N, Rasmussen J, Lund E. A Method of "Exact" Numerical Differentiation for Error Elimination in Finite-Element-Based Semi-Analytical Shape Sensitivity Analyses. Mech. Struct. & Mach. 1993;21(1). Then the surface-based optimized shape can be fixed. Afterwards, the composite laminate patches are laid-out on the surface of the object and the angles of each ply or patch are optimized, as discussed in Bruyneel M, Fleury C. Composite structures optimization using sequential convex programming. Advances in Engineering Software. 2002;33, and Johansen L, Lund, E. Optimization of laminated composite structures using delamination criteria and hierarchical models. Struct Multidisc Optim. 2009;38. Then the thickness of each laminate or sandwich patch can be optimized simultaneously, as discussed in Pedersen P. On thickness and orientational design with orthotropic materials. Struct Optim. 1991;3., with the angles or in yet another optimization step for just the thickness, as discussed in Sjølund JH, Lund E. Structural gradient-based optimization of wind turbine blades with fixed outer geometry. Composite structures. 2018;203. An overview over both gradient based methods (also called sensitivities-based methods) and gradient-less methods for the previous various optimization steps can be found in Sjølund JH, Lund E. Structural gradient-based optimization of wind turbine blades with fixed outer geometry. Composite structures. 2018;203, Ghiasi H, Pasini D, Lessard L. Optimum stacking sequence design of composite materials Part I: Constant stiffness design. Composite Structures. 2009;90(1), and Ghiasi H, Fayazbakhsh K, Pasini D, Lessard L. Optimum stacking sequence design of composite materials Part II: Variable stiffness design. 2010;93(1).

These optimization steps can be individually robust and accurate, but the global result can be far from optimal because the sets of parameters controlling the object shape and material properties are optimized using separate sequential steps. In other words, a truly optimal topology or shape might become sub-optimal once the material anisotropy is considered.

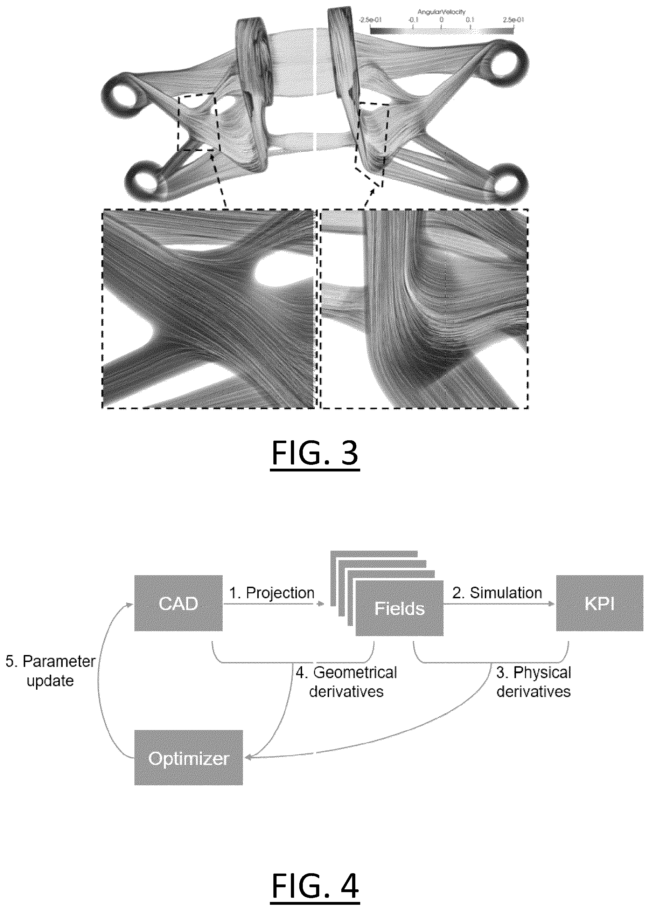

Another problem is the lacking CAD representation when directly optimizing many low-level variables for the density and anisotropy of the material throughout the design domain in an extended topology optimization framework (for example as discussed in https://www.researchgate.net/publication/353194654_Large-Scale_Three-Dimensional_Aniso tropic_Topology_Optimization_of_Variable-Axial_Lightweight_Composite_Structures). These approaches offer maximum design freedom in the design variable description for the optimization. Thus, theoretically they achieve high performance structural designs. The drawback is that the final optimized structure may be difficult to reinterpret into a CAD model or may not be manufacturable due to the complicated shape and material orientations present in the final optimized structure. This is illustrated on FIG. 3 which shows a screenshot of result obtained by such approaches, with a zoom on these complicated shapes.

The reference Gandhi Y, Minak GA. Review on Topology Optimization Strategies for Additively Manufactured Continuous Fiber-Reinforced Composite Structures. Appl. Sci. 2022;12. gives an additionally overview over topology optimization approaches including composite design variables. The following approaches are the most common ones:

- Topology optimization using the so-called density approach including composite design variables and sensitivity calculation is for example given in references Xu Y, Zhu J, Wu Z, Cao Y, Zhao Y, Zhang W. A review on the design of laminated composite structures: constant and variable stiffness design and topology optimization. Adv Compos Hybrid Mater 2018;1(3):460, Hvejsel CF, Lund E. Material interpolation schemes for unified topology and multimaterial optimization. Struct Multidisc Optim 2011;43(6), and Hozic D, Thore C, Cameron C, Sahbi Loukil M. A new method for simultaneous material and topology optimization of composite laminate structures using Hyperbolic Function Parametrization. Composite structures 2021;276. The so-called topology optimization density approach does not contain any CAD information and the composite design variables are not coupled to a CAD description.

- The references Allaire G, Delgado G. Stacking sequence and shape optimization of laminated composite plates via a level-set method. J Mech Phys Solids 2016;97, and Yanan X, Yunkai G, Chi W, Jianguang F, Guang-yong S, Grant P S, Qing L. Concurrent optimization of topological configuration and continuous fiber path for composite structures - A unified level set approach. Computer Methods in Applied Mechanics and Engineering 2022;399 also combine topology optimization and composite design variables but apply the so-called level-set topology optimization. Again, none of these level set topology optimization approaches contain any CAD information. Additionally, none the examples in these references use a 3D continuum modeling which would be required for a general 3D coupling.

- The work in references Smith H, Norato J. Topology optimization with discrete geometric components made of composite materials. Computer Methods in Applied Mechanics and Engineering. 2021;376, Smith H, Norato J. Topology optimization of structures made of fiber-reinforced plates. Struct Multidisc Optim 2022;65, and Greifenstein J, Letournel E, Stingl M, Wein F. Efficient spline design via feature-mapping for continuous fiber-reinforced structures. 2023;66. applies the moving morphable components (MMC) approach for optimizing strictly bar, strictly plate, and strictly piecewise linear splines structures for orthotropic fiber-reinforced material, respectively. Thus, these MMC approaches for the orthotropic fiber-reinforced materials do not disclose CAD descriptions but just parametric descriptions of simple bar and beam members for designing.

<Also known is US 2023/274048 A1 which discloses a method based on fatigue damage sensitivity computations.

Also known is US 2023/114354 A1 which discloses a method for designing a modeled object representing a mechanical part formed in a material having an anisotropic behavior with respect to a physical property.>

Within this context, there is a need for an improved method for optimization of one or more anisotropic material properties of a composite part.

## SUMMARY

<The invention is defined by the appended claims.>

## BRIEF DESCRIPTION OF THE DRAWINGS

Non-limiting examples will now be described in reference to the accompanying drawings, where:

- FIG.s 1 to 15 illustrate the method; and

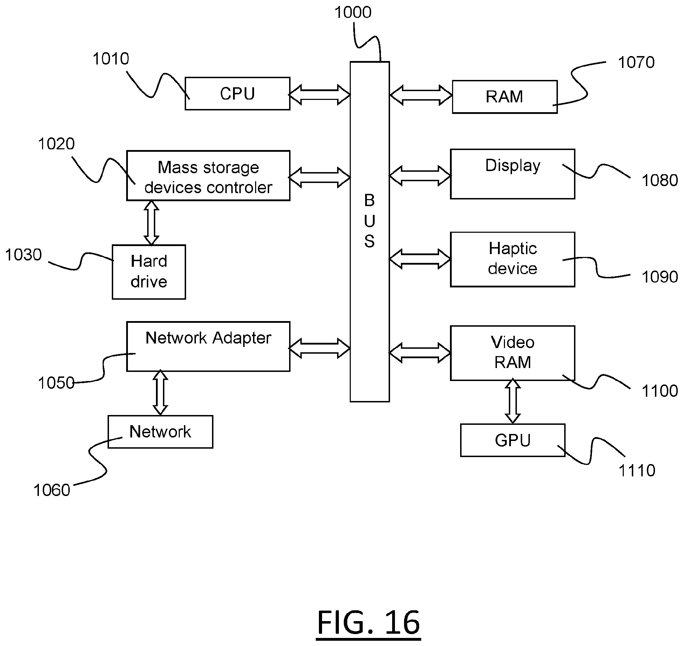

- FIG. 16 shows an example of the computer system.

## DETAILED DESCRIPTION

It is provided a computer-implemented method for optimization of one or more anisotropic material properties of a composite part. The method comprises providing a CAD model. The CAD model represents the composite part. The CAD model includes a feature tree having one or more CAD parameters. Each CAD parameter has an initial value. At least one CAD parameter influences the one or more anisotropic material properties of the composite part. The method further comprises providing an optimization program. The optimization program is specified by one or more use and/or manufacturing performance indicators. The one or more indicators comprise one or more objective functions and/or one or more constraints. The method further comprises modifying the initial values of the one or more CAD parameters by solving the optimization program using a gradient-based optimization method. The optimization method has as free variable the one or more CAD parameters. The optimization method uses sensitivities. Each sensitivity is an approximation of a respective derivative of a respective performance indicator with respect to a respective CAD parameter. The sensitivities are compositions of:

- approximated respective derivatives each of a respective performance indicator with respect to one or more material property fields, each material property field of the one or more material property fields representing a distribution of an anisotropic material property of the one or more anisotropic material properties, and
- approximated respective derivatives each of the one or more material property fields with respect to a respective CAD parameter.

The method constitutes an improved solution for optimization of one or more anisotropic material properties of a composite part.

The method indeed optimizes one of more CAD parameters of a CAD model with respect to the one or more performance indicators, at least one of these parameters influencing the one or more anisotropic material properties of the composite part. This means in other words that the method optimizes these one or more anisotropic material properties of the composite part, through optimization of the CAD parameter that influences them, for achieving optimal values of the one or more performance indicators. The method thus results in (*i.e.* outputs, for example as CAD files) CAD models of composite parts having one or more anisotropic material properties (*e.g.* stiffness, thermal conductivity) which are optimally designed for the composite part to optimize the performance indicators, *i.e.* to exhibit optimized performances with respect to these indicators. In other words, not only does the method optimize the shape of the part (since the CAD parameters influence the shape), but also its anisotropic physical properties (*e.g.* fiber orientations) since at least one optimized CAD parameter influence(s) them. Each performance indicator relates to use of the part (for example response to a particular load during use, mass, or stiffness) or to manufacturing of the part (for example the indicator captures constraints and/or tolerances of the manufacturing process or machine and the part is optimized to meet those). The method may comprise both performance indicators for use (*e.g.* mass, stiffness, response to loads) and performance indicators for manufacturing (*e.g.* constraints for enforcing manufacturability of the output model, *e.g.* by a specific manufacturing process).

Furthermore, the method optimizes a CAD model (*i.e.* optimizes the CAD parameters thereof) of a composite part. This means that the output of the model is an optimized CAD model, *i.e.* which has its CAD parameters' values optimized with respect to the optimization problem handled by the method. Such model can directly be used for manufacturing the composite part, *i.e.* represents a manufacturable object, and its CAD parameters allow to entirely define the specifications of the manufacturing process. The output optimized CAD model may for example directly serve (*e.g.* in the form of a CAD file, or for example after conversion into a corresponding CAM (computer-aided manufacturing) file) to control and/or set the manufacturing process and/or machine(s)/tool(s) thereof, as further discussed hereinafter Thus, contrary to existing optimization methods that work on CAE models (*e.g.* finite element meshes) and that, due to the freedom that they offer to the search for optimal values in the optimization, may lead to optimized part which are in practice too complex to be manufactured (notably the optimized orientations of the physical properties of the materials may be in practice unmanufacturable), the output of the method, being a CAD model, is directly usable as a file to control and/or set the manufacturing process for manufacturing the optimized part that corresponds to the CAD model. Furthermore, as said above, manufacturability can be enforced by having one or more performance indicators implementing one or more manufacturing constraint(s). Alternatively, manufacturability may be enforced without using dedicated performance indicators (which for some manufacturing contexts may be unfeasible), but may be enforced by using appropriate CAD parameters in the model (which may or may not be part of the parameter that are optimized by the method). This further illustrates the technical advantage of directly optimizing a CAD model: manufacturability is guaranteed and can be enforced by CAD parameters. For example, the at least one CAD parameter influencing the one or more anisotropic material properties of the composite part may satisfy (or capture) a constraint of a manufacturing process for manufacturing the composite part.

The method thus targets the physical and/or geometrical optimization of 2D or 3D objects having anisotropic/-composite/microstructure/fibered material modeled in CAD software. A key characteristic of these CAD models is that they can be designed geometrically exactly and unambiguously by chaining a small number of high-level heterogeneous parameterized design operations (sketch, extrude, chamfer, loft volumes, shell offsets, fillet, pocket, trim, emboss, transformations, multi-instantiation, assemblies, Boolean operations) and edited by modifying their high-level geometrical numerical parameters. Note that this is the main distinction compared to the simpler polyhedral representations such as a triangular surface mesh, which can represent most shapes but do not provide modification or parameterization capabilities required in an industrial design and manufacturing context.

The proposed method solves the following problems:

- handle a large number of design variables (thus traditionally excluding gradient-less methods);
- handle heterogeneous parameters directly being present in the representation for the CAD construction;
- model both shape and anisotropic material properties in a single common representation;
- map between the high-level CAD parameters and the low-level local material behavior modeling the mechanical properties and physical performance.

As discussed hereinbelow, the method may solve the previously mentioned challenges by projecting the CAD model containing the components for the anisotropic representations into a continuous Eulerian representation. The local properties such as density, orientation or fiber fraction can be captured by scalar fields on a volumetric mesh. Then, the finite element analysis simulates the physical behavior of these scalar fields and evaluate the Key Performance Indicators (KPI). The mathematical derivatives of the simulation and projection steps are evaluated separately and combined using the chain rule to obtain the gradients of the physical performance KPIs with respect to the CAD parameters applied to iteratively optimize the model.

The proposed method and its implementations offer the following benefits:

- It directly modifies the heterogeneous parameters defined in the CAD representation. Thus, removing the need for a CAD reinterpretation step of the optimization results. Additionally, it guarantees the preservation of the properties for manufacturability in the original CAD model.
- It enables the use of efficient iterative gradient-based optimization schemes. Thus, allowing computational efficiently to optimize for many CAD parameters. Additionally, it also allows many constrains for the various performance indicators (KPIs) where the constraints can both be active and inactive during the iteratively optimization iterations.
- It optimizes simultaneously both shape and material properties as a unified coupled problem and therefore, achieves a better optimum than solving a sequence of multiple de-coupled optimization sub problems.

The method is for optimization of one or more anisotropic material properties of a composite part. The method thus outputs a composite part design having such optimized anisotropic material properties. Specifically, the method takes as input a CAD model of such part with CAD parameters, at least one of which influencing the one or more anisotropic material properties of the composite part, and modifies their values (thus, in particular, the values of the parameter(s) influencing the anisotropic material properties) to reach (*e.g.* up to a convergence criterion) optimal value(s) for the performance indicator(s). The output of the method is thus the optimized CAD model, *i.e.* the CAD model with these optimized values for the CAD parameters, including those parameters influencing the anisotropic material properties.

The method thus forms a design step (specifically an optimization step of a design process, where already existing CAD parameters are optimized) and is thus for designing a mechanical product/part, namely the composite part. "Designing mechanical part/mechanical product" designates any action or series of actions which is at least a part of a process of elaborating a modeled object (3D or 2D) of the mechanical part/mechanical product. The method is an optimization step within this process.

The composite part is a part formed in a composite material. A composite material is a material which is produced from two or more constituent materials. These constituent materials have notably dissimilar chemical or physical properties and are merged to create a material with properties unlike the individual elements. Within the finished structure, the individual elements remain separate and distinct, distinguishing composites from mixtures and solid solutions. The composite part has anisotropic material properties, *i.e.* the properties of the composite material are directionally dependent. The composite material may be an orthotropic material, that is an anisotropic material where there are three mutually orthogonal planes of symmetry in the material properties. The one or more anisotropic material properties may consist of several anisotropic material properties. Each anisotropic material property may also be referred to as "material anisotropic property type", such that each property of the one or more material anisotropic properties is a respective type of material anisotropic property.

The method comprises providing the CAD model representing the composite part and the optimization program. The provided CAD model and the optimization program thus form inputs of the method. The provided CAD model may be referred to as "the input CAD model". The provided CAD model is a feature-based CAD model that includes a feature tree. The concept of feature-based CAD model is known *per* se from CAD and is further discussed hereinafter. Likewise, the concept of feature-tree is well-known from CAD. The feature tree is a tree organization of CAD operations (*e.g.* including Boolean and non-Boolean operations) on CAD features that each model a portion of the composite part. More specifically, the feature tree is a directed acyclic graph (as discussed in https://en.wikipedia.org/wiki/Directed_acyclic_graph, that describes/captures the order of sequence and combination of the CAD operations. As known *per se* from CAD, a CAD feature includes shape/geometry information and parametric information for representing a portion of the composite part modeled/captured by the CAD feature. The CAD feature may as known comprise a definition/specification of a geometry/-shape corresponding to the portion modeled by the CAD feature (*e.g.* a definition/specification geometric primitive) and one or more CAD parameters that specify the geometry and its topology. The feature tree thus includes one or more CAD parameters each being respective to a respective CAD feature of the feature tree. Non-limitative examples of such CAD parameters include thickness of a plate, depth of a pocket, angle of a fibered ply, control points of a subdivision surface. One or more of these CAD parameters influences the anisotropic material properties of the composite part.

A CAD parameter that influences the anisotropic material properties of the part is a CAD parameter of which value, when changed, modifies at least one anisotropic material property of the part (*e.g.* all such properties). Non limiting examples of such parameters include the angle of a fibered ply, or a CAD variable controlling the profile of a wind turbine blade (it affects the shape of the cross-section bit also the material orientations in the composite laminates since these are conformal to the shape and follow its curvature), or any CAD parameter that affects the orientation of the material of the composite part.

The model may comprise at least one CAD parameter influencing the one or more anisotropic material properties of the composite part and that captures a constraint of a manufacturing process for manufacturing the composite part. This allows to optimize the design for a specific manufacturing process. Alternatively or additionally, such constraint may be captured by a constraint of the optimization program that implements a manufacturing performance indicator, which also allows to optimize the design for a specific manufacturing process.

The CAD model may be a 2D CAD model (*i.e.* forming a 2D CAD representation of the composite part) or a 3D CAD model (*i.e.* forming a 3D CAD representation of the composite part). The CAD model may include, besides the feature tree, a 2D geometrical representation (if the model is 2D) or 3D geometrical representation (if the model is 3D), for example a B-rep obtained by executing the feature tree. The geometrical representation may be or include an outer surface geometrical representation of the composite part. The providing of the method may comprise providing a mesh (*i.e.* 2D or 3D) comprising a geometrical representation, for example and outer surface geometrical representation, of the model.

The input CAD model may stem from a previous design process (*e.g.* performed on another system or software, *e.g.* during another design session). Providing the CAD model may comprise downloading the CAD model from a memory or server on which the CAD model has been stored further to its design. Alternatively, providing the CAD model may comprise designing the CAD model. Designing the CAD model may comprise designing the CAD model from scratch on a CAD system, *e.g.* by iteratively building the feature tree. Alternatively, designing the CAD model may comprise designing first a CAE model (*i.e.* a finite element model) representing the composite part on a CAE system, using any CAE software solution, and converting the CAE model into a CAD model using any CAD-to-CAE conversion method, and optionally then storing the CAD model on a memory or server. Providing the CAD model may yet comprise downloading an already designed CAE model, *e.g.* from a memory or server on which the CAE model has been stored further to its design, and then converting the CAE model into the input CAE model.

Providing the optimization program may comprise downloading the optimization program from any memory or server where the program is available. Alternatively, providing the optimization program may comprise launching any software where the program is implemented. Yet alternatively, providing the optimization program may comprise coding/programming the optimization program or downloading (and optionally modifying) an already coded/programmed optimization program.

The optimization program comprises an optimization algorithm to solve an optimization problem. The optimization problem refers to a mathematical optimization problem that consist in minimizing or maximizing one or more objective functions, optionally under one or more constraints, or minimizing or maximizing any other quantity under the constraint(s). The optimization program is thus specified by one or more use and/or manufacturing indicators that comprise the objective function(s) and/or the constraint(s). The optimization program comprises an optimization algorithm that performs such a minimization or maximization. The optimization program may be designed so that data about the optimization may be inputted by a user prior to launching the optimization program, said data including the objective function(s), the constraint(s), any convergence threshold, and/or any maximal value of iterations steps for the algorithm. The method may comprise a step of a user providing at least a part of such data, prior to the optimization step. The optimization program may also be designed so that a user may select an optimization method to perform the optimization. The method may comprise a step of a user selecting the optimization method. The selection of the optimization method may be constrained to a predefined list of optimization methods, *i.e.* the user may only select a method within this list. The list may consist in gradient-based optimization methods only, as the optimization method used by the method is a gradient-based optimization method. The concept of gradient-based optimization method, also referred to as "gradient method", is well known (see for example https://en.wikipedia.org/wiki/Gradient method, ). The gradient-based optimization method used by the method may be any one of the methods of the following list (which may form the previously-discussed list for a user-selection): gradient descent, stochastic gradient descent, coordinate descent, Frank-Wolfe algorithm, Land-weber iteration, random coordinate descent, conjugate gradient method, derivation of the conjugate gradient method, nonlinear conjugate gradient method, biconjugate gradient method, biconjugate gradient stabilized method, and method of moving asymptotes.

The one or more use and/or manufacturing performance indicators are data that define the underlying optimization problem, *i.e.* the optimization problem amounts to optimize the performances of the composite part represented by the CAD model with respect to this/these performance indicator(s). A performance indicator is in other words data (*e.g.* a mathematical expression, such as a mathematical function or constraint (*e.g.* captured by an equation or a system of equations) that measure a performance to be achieved and/or respected by the composite part represented by the CAD model, once manufactured. The performance is with respect to use and/or manufacturing, *i.e.* the performance is a physical performance (*e.g.* a behavior) of the composite part during use and/or during manufacturing, as the indicator is a use and/or manufacturing performance indicator. A performance of the product during use is a physical behavior of the part with respect to a given use of the product, such as a thermal behavior of the part, an aerodynamic behavior of the part, or a structural behavior of the part, *e.g.* when the part is subject to one or more loads (*e.g.* thermal forces, structural loads, or fluid flows). A performance of the part during manufacturing is a physical behavior of the part with respect to a given manufacturing process of the part, such as a compliance of the part with constraints of the manufacturing process and/or with specifications of a manufacturing tool carrying out the process.

The one or more performance indicators comprise one or more objective function. An objective function is herein a function of which value is to be optimized (*i.e.* minimized or maximized) by the optimization. The objective function measures a performance of the part with respect to use and/or manufacturing of the product, the function having as variable the CAD model or one or more CAD parameters. The function outputs a value that is large when the performance is achieved, in which case the function is to be maximized, or a value that is small when the performance is achieved, in which case the function is to be minimized.

The one or more indicators performance may additionally or alternatively comprise one or more constraints. A constraint is herein a mathematical expression (*e.g.* an equation or a set of equations, or a fixed value of a parameter) that is to be respected during the optimization. The constraint captures a performance that the composite part is to fulfill (that is, mandatorily), such as a prescribed total volume of material of the product, or respect of tolerances of the manufacturing process or manufacturing tool. The optimization may optimize the objective function(s) under the constraint(s), *i.e.* the optimization aims at finding the variable values that optimize the objective function(s) while fulfilling the constraint(s).

Examples of performance indicators (also referred to as KPI's) in mechanical design and that may be involved in the method are: maximize stiffness, minimize mass, minimize stress, maximize heat transfer coefficient, optimize eigenmodes).

The method may comprise, *e.g.* at the providing of the optimization program, *e.g.* by a user, selecting the performances indicators to optimize, *e.g.* from a list, the selection yielding the objective function(s) and/or constraint(s) for the optimization.

The CAD parameters values may be denoted $CAD_p \, \forall p \in \Omega_{param}$, $\Omega_{param}$ being the set of CAD parameters. The performance indicators may be referred to as "Key Performance Indicators" and their values may be denoted $KPI_n \, \forall n \in \Omega_{perfo}$, where $\Omega_{perfo}$ is the set of performance indicators.

Further to the providing of the inputs, the method comprises modifying the initial value of the one or more CAD parameters. The modification of the initial value of the one or more CAD parameters may be referred to as the optimization of the one or more CAD parameters. Indeed, this step finds or tends to find the value(s) of the one or more CAD parameters, which are free variables of the optimization, that optimize the one or more objective function(s) and/or that fulfill the one or more constraints. Modifying the initial values thus consist in an optimization that solves the optimization program, *i.e.* that runs the optimization algorithm of the optimization program to solve the underlying optimization problem.

The optimization uses a gradient-based optimization method, as previously discussed. The optimization method has as free variables the one or more CAD parameters of which values are to be modified to perform the optimization, and uses sensitivities of the performance indicators with respect to these variables (*i.e.* derivatives approximations) to perform the optimization, since the method is gradient-based, as known *per se* in the field of optimization. In other words, the CAD parameters are the free variables of the optimization that the optimization method can modify (*i.e.* the optimization can modify the values of the CAD parameters) to optimize the performance indicators, and the sensitivities correspond to partial derivatives of the indicators to optimize with respect to the free variables. It is to be understood that, when saying that the values of the one or more CAD parameters are modified by the optimization, it may be that the values are not modified for all the CAD parameters. For example, some CAD parameters may have their values set fixed, and the others, including or consisting in those that influences the anisotropic material properties, have values which are the free variables of the optimization. In this case, the method may comprise, prior to the optimization, selecting (*e.g.* by a user) the CAD parameters for which their values are free variables, the values of the other CAD parameters remaining then fixed during the optimization. Alternatively, all the values of all the parameters may be free variables of the optimization. The method may include, prior to updating the CAD parameters with the optimization method, a step of computing the sensitivities and/or the performance indicators.

The sensitivities are compositions of:

- approximated respective derivatives each of a respective performance indicator with respect to one or more material property fields, each material property field of the one or more material property fields representing a distribution of an anisotropic material property of the one or more anisotropic material properties, and
- approximated respective derivatives each of the one or more material property fields with respect to a respective CAD parameter.

Each material property field is respective to one of the material properties, *i.e.* there is one respective material property field for each respective material property. Each material property field is a distribution of scalar values, also referred to as material property values, or local material property values, and represents a distribution of the anisotropic material property (*e.g.* material orientation, heat conductivity) to which the field is respective. For example, for material orientation, the local property value may be the local XY (in 2D) or XYZ (in 3D) coordinates of the material orientation. The distribution of the material property values is on a mesh (2D or 3D mesh) of a space (2D or 3D) that includes a geometrical (*e.g.* outer surface) representation of the composite part. The mesh may for example be a mesh of volumetric or surface elements (*e.g.* the elements being tetrahedral/triangular, hexahedral, or regular cubes/squares). For each material property field, each property value is respective to a respective element of the mesh and indicates a value of the property at this element. Each value of a given material property at each element of the mesh may be referred to as a local material property value (*i.e.* of the given material property), since it pertains to the value of the material property value at the element.

In implementations using fibered material(s), such as use cases discussed hereinbelow, the local property fields $M$ are encoded to have 4 values per element (corresponding to 4 material anisotropic properties, or property types): 1 value for the material density, 2 values for the rotational angles of the fiber orientation in 3D, and 1 value for the ratio of fiber (i.e. ratio of anisotropic fiber to isotropic material where the isotropic material frequently models the binding "glue" or "matrix" in which the fibers are manufactured).

The CAD model may be formed by one or more CAD components. Each CAD component corresponds to a region of space, *i.e.* that forms a portion of the CAD model. In this case the method comprises computing one or more signed distance fields each to a respective CAD component. For each material property field, each local material property value of the field results from a weighted combination of material properties of the components of the CAD model weighted by their signed distances to the local material property value. The one or more components may consist of several components.

Each signed distance field is respective to a CAD component. The concept of signed distance field is known. A signed distance field is a distribution of signed distances with respect to a certain geometry. In this case, each signed distance field is respective to a component and forms a distribution of signed distance values, each respective to an element of said mesh and representing a signed distance between this element and the CAD component (or, its geometry representation, *e.g.* outer surface representation, on the mesh). In other words, the signed distance field respective to a component is a distribution of signed distances values on said mesh to the component, the mesh including a geometric (*e.g.* outer surface) representation of the component (which may be part of the geometric representation of the whole composite part). Each signed distance value in this distribution is respective to an element of the mesh and indicates a value of a distance (*e.g.* Euclidean distance, Manhattan distance, pixel distance) of this element to the (*e.g.* outer surface) representation of the component, the value having a sign when the element corresponds to an inside portion of the component and the opposite sign when the element corresponds to an outside portion of the component.

Each local material property value of the field results from a weighted combination of material properties of the components of the CAD model weighted by their signed distances to the local material property value. For example, for each local material property, and for each element of the mesh, the local material property value at this element is equal to a weighted combination of the material properties values of the components each weighted by the signed distance between the element and the component, or by a projection of such distance as explained below. The weighted combination may be a weighted sum.

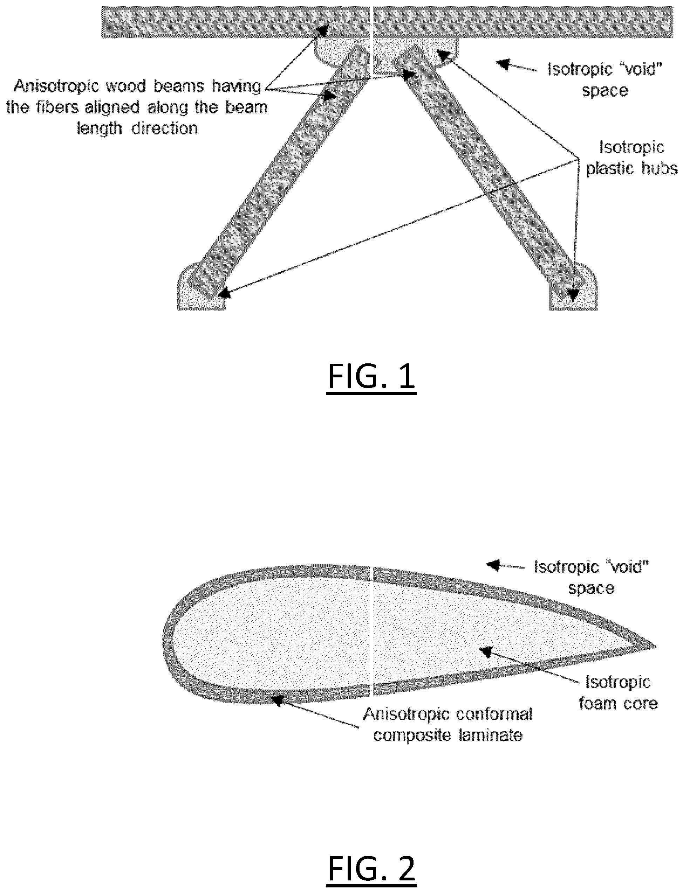

The method may comprise, *e.g.* prior to updating the CAD parameters with the optimization method, computing the signed distance field(s) and the material property field(s), as further discussed hereinbelow. This projects the CAD model representation (*i.e.* on the mesh) into a collection of scalar fields encoding local material properties for each element of the mesh. As known, the CAD model includes a geometrical boundary representation on the mesh, which means that it defines a solid object by hierarchically separating space into regions or components having different material properties (see again FIG.s 1 and 2), *e.g.* solid/empty, steel, wood, plastic, concrete, carbon fiber laminates, air, void. The projection defines the local material properties of each volumetric element in the mesh as a quantity of the material properties of nearby components weighted by a nonlinear distance metric (the signed distance). For example, referring to the use case shown in FIG. 2, by this process, an element in the foam core component will receive low-stiffness isotropic material properties. Similarly, an element at the interface between the composite laminate and foam core components will receive a mixture of low stiffness isotropic material properties and high stiffness anisotropic material properties aligned with the local orientations of the composite laminate. Mathematically, this projection is obtained by calculating a linear Signed Distance Field (SDF) to the components $\Omega_{comp}$ of the tessellated representation of the CAD model over the elements of the mesh $\Omega_{mesh}$ as known in the art (see https://www2.imm.dtu.dk/pubdb/edoc/imm1289.pdf,

$$SDF_{ij}(CAD),$$

$\forall\ i \in \Omega_{mesh}, j \in \Omega_{comp}$
where $i$ denotes an element of the mesh $\Omega_{mesh}$ of the CAD model, and $j$ denotes a component of the set of components $\Omega_{mesh}$, and $SDF_{ij}(CAD)$ denotes the signed distance field value between element $i$ and component $j$.

Each weight may correspond to (*e.g.* be equal to) a projection of a signed distance value of a component by a function that maps $]-\infty; +\infty[$ onto $[0; 1]$ and that has well-defined first order derivatives. The function may be a smooth nonlinear Heaviside projection. The smooth nonlinear Heaviside function controls the smearing of the material properties (see https://en.wikipedia.org/wiki/Heaviside step function,). Any positive monotonous smooth approximation of the Heaviside function can be used for this smearing step instead of the Heaviside function. The weights $H_{ij}$ may be given by the following formula (using smooth nonlinear Heaviside):

$$H_{ij} = \frac{1}{1 + e^{\frac{SDF_{ij}}{0.2\alpha \times 2l}}},$$

$\forall\ i \in \Omega_{mesh}, j \in \Omega_{comp}$
where $l$ is the size of the mesh elements and $\alpha$ is the desired smearing distance, which may equal 2 in implementations.

For each material property type $k \in \Omega_{type}$ (e.g. material density, fiber azimuth angle, fiber elevation angle, fiber/glue ratio, fiber stiffness, glue stiffness), the local material property values for each element $i$, may be given by the formula:

$$M_{ik} = \frac{\sum_{j \in \Omega_{comp}} H_{ij}\, m_{ijk}}{\sum_{j \in \Omega_{comp}} H_{ij}}$$

$\forall\ i \in \Omega_{mesh}, \forall\ k \in \Omega_{type}$
where $M_{ik}$ is the local material property value at element $i$ of mesh $\Omega_{mesh}$ for the property type $k$ of the CAD model, $j$ is a component, $\Omega_{comp}$ is the set of all the components, $H_{ij}$ are the weights, and $m_{ijk}$ are the material properties of the components.

It is to be noted that in the above, $m_{ijk}$ depends on $i$ (the index of the element) because such dependence may exist in physical reality. For example, if component $j$ is a sheet of composite material glued on the surface of a part, the fiber angles in the composite vary in space within the component (it follows the tangent to the surface), so it depends indeed on $i$. In other words, within a component $j$, property $k$ may vary from an element $i$ to another.

Different functions for the local properties $M_{ik}$ being a function of the Heaviside function $H_{ij}$ may be alternatively used. Optionally, the above formula may be modified to enforce bounds or additional scaling upon the local properties if the direct determined local properties are exceeding their feasible physical range.

In implementations using fibered material(s), such as use cases discussed hereinbelow, the local property fields M are encoded to have 4 values per element *(i.e.* k=1...4): 1 value for the material density, 2 values for the rotational angles of the fiber orientation in 3D, and 1 value for the ratio of fiber (i.e. ratio of anisotropic fiber to isotropic material where the isotropic material frequently models the binding "glue" or "matrix" in which the fibers are manufactured). However, it is possible to use a single, few or more values per element depending on the type and characteristics of the anisotropy being employed. Alternative implementations may use many values as there are independent coefficients in the constitutive material being considered: (see https://pkel015.connect.amazon.auckland.ac.nz/SolidMechanicsBooks/Part I/BookSM Part I/06 LinearElasticity/06 Linear Elasticity 03 Anisotropy.pdf,

As previously said, the computation of the signed distance fields and material property fields, for example by computing the above formulae for the $M_i$ and $H_{ij}$, may form a step of the method that precedes the modifying of the values of the CAD parameters to iteratively modify the CAD model in an optimization loop until some convergence criterion, number of optimization iterations, runtime, and/or KPI score is reached. This step of computation of the fields may be referred to as "projection". FIG. 4 shows a flowchart of implementations of the method, or more specifically, of the steps that follow the step of providing the inputs. The flowchart of FIG. 4 shows the projection step, which is the first step in the flowchart.

Further to the projection/computation of the fields, and still prior to the update of the CAD parameters' values, the method may comprise a step, which may be referred to as "simulation" (illustrated by the second step in the flowchart of FIG. 4), of computation of the performance indicators, also referred to as KPIs as previously said. The method may compute some KPIs, for example the mass of the part, by summing up values of these KPIs for each element of a given partition of the part, *e.g.* the mass of each element for a given partition or the entire structure. Other KPIs are more complex to compute, and the method may compute them by simulating the behavior of the composite part using finite element modeling as known in the art. For example, computing the deformation of the part under load scenarios may involve assembling the local stiffness matrices of each element into a global stiffness matrix $K$ of the entire mesh and subsequently solving for the primal solution of the equilibrium given by the state equation:

$$Ku = f$$

where $f$ is the known nodal force vector and $u$ is the unknown nodal displacement vector. The method may use appropriate simulation methods for performing this computation. Computing the KPIs thus uses known methods, be it direct analytical computations or simulations, and thus need not being further discussed.

The optimization that modifies the values of the CAD parameters uses the sensitivities. The method may thus, prior to updating the CAD parameters values, computing the sensitivities, which as said above are approximations of derivatives.

The method may for example comprise a step, further to the simulation step but prior to the update of the CAD parameters, of determining the approximations of the derivatives of the KPIs with respect to the local property values of each element:

$$\frac{\partial KPI_n}{\partial M_{ik}},$$

$\forall n \in \Omega_{perfo}$, $i \in \Omega_{mesh}$, $k \in \Omega_{type}$.
This may be done by using known methods.

For example, for the stiffness KPI, an element in an isotropic component would have an orientation derivative value practically equal to zero. Thus, exposing the fact that the local angle of the present element has no impact on the deformation of the structure since this particular element is isotropic. Note, the derivatives would not be exactly zero because a strictly positive monotonous smooth Heaviside function in the projection step is purposefully used. In contrast, the local angle of an element in the composite laminate region of the structure could have a strong impact on the structural deformation for a given load scenario, which would be characterized by large derivative values.

As continuous field representation of the part is used, these derivatives $\frac{\partial KPI_n}{\partial M_{ik}}$ may be calculated efficiently by the method using the adjoint method as described in https://www.researchgate.net/publication/342871596 Structural topology optimization with smoothly varying fiber orientations, Using the adjoint method contributes to the computational performance of the approach disclosed in the present disclosure. The reason is that for the adjoint method, the number of calls to solve the computational expensive linear system scales with the size of $\Omega_{perfo}$ (which is typically less than 10) instead of scaling with the size of $\Omega_{mesh}$ (which is typically more than 100 000). The computation of the approximations of the derivatives $\frac{\partial KPI_n}{\partial M_{ik}}$ may be referred to as a step of computing the physical derivatives, illustrated by step 3 on FIG. 4.

The method may also comprise a step, still further to the simulation step but prior to the update of the CAD parameters, of determining the approximations of the derivatives $\frac{\partial M_{ik}}{\partial CAD_p}$ of the property fields $M_{ik}$ with respect to the CAD parameters $CAD_p$ :

$$\frac{\partial M_{ik}}{\partial CAD_p},$$

$\forall i \in \Omega_{mesh}, k \in \Omega_{type}, p \in \Omega_{param}.$
These derivatives capture how each CAD parameter affects the local properties of each element in the mesh. This step enables the coupled optimization of CAD parameters controlling both the shape and local material properties of the composite part.

For example, a CAD variable controlling the profile of a wind turbine blade will affect the shape of the cross section but also affect the material orientations in the composite laminate since these are conformal to the shape and follow its curvature. These derivatives may be evaluated using the finite difference method. This is achieved by sequentially applying a small perturbation $h_p$ to each CAD parameter and observing the impact on the material properties values as follows:

$$\frac{\partial M_{ik}}{\partial CAD_p} \cong \frac{M_{ik}\left(CAD_p + \frac{h_p}{2}\right) - M_{ik}\left(CAD_p - \frac{h_p}{2}\right)}{h_p},$$

$\forall i \in \Omega_{mesh}, k \in \Omega_{type}, p \in \Omega_{param}$
where $CAD_p$ is the respective CAD parameter, where $h_p > 0$ is a small perturbation, where $\Omega_{param}$ is a set of the CAD parameters.

The above formulation uses a central finite difference scheme, but forward or backward schemes may alternatively be used for the finite difference. The finite difference approximation is very stable and reliable for this purpose. This is explained by the fact that each CAD parameter typically affect the values of many mesh elements since $\Omega_{param} \ll \Omega_{mes}$ for practical and industrial applications. Therefore, numerical inaccuracies of the finite difference approximations will cancel-out when aggregating the contributions of multiple elements onto the CAD parameters. Notice, that the analytical derivatives of the CAD system are intractable for any non-trivial CAD model, hence the benefit to use an approximation of the derivatives.

The computation of the approximations of the derivatives $\frac{\partial M_{ik}}{\partial CAD_p}$ may be referred to as a step of computing the geometrical derivatives, illustrated by step 4 in FIG. 4.

The geometrical and physical derivatives, once computed, may be then combined using the chain rule. In other words, each sensitivity $\frac{\partial KPI_n}{\partial CAD_p}$ is of the type:

$$\frac{\partial KPI_n}{\partial CAD_p} = \frac{\partial KPI_n}{\partial M_{ik}} \frac{\partial M_{ik}}{\partial CAD_p},$$

$\forall n \in \Omega_{perfo}, p \in \Omega_{param}$
where $\Omega_{perfo}$ is the set of performance indicators.

The method may then perform the optimization using the gradient-based optimization method (e.g. first-order gradient-based optimization scheme, such as Gradient Descent, Method of Moving Asymptotes, Augmented Lagrangian, Adam, LBFGS) to modify (update) the values of the CAD parameters to tend to reach an optimal value of the KPI, as known in the art. This may be referred to as "parameter update", illustrated at step 5 on FIG. 4.

The steps 1 to 5 (referring to FIG. 4), *i.e.* the computation of the fields (projection step), the computation of the KPIs (simulation step), the computation of the approximations of the derivatives (geometrical and physical), and the update of the CAD parameters may be iterated, where the CAD parameters resulting from the update (step 5) are used as input to the projection step (step 1) of the next iterations. In other words, the method may comprise the step of providing the inputs, and then the step of modifying the values of the CAD parameters by applying the gradient-based optimization method, which may consist in iterations of said step 1 to 5 where the optimization method is used on step 5 to update the parameters based on the sensitivities computed as a result of the previous steps 1-4. This is illustrated on FIG. 4. This iterative process may converge in 10 to 100 iterations due to the use of gradient-based optimization.

The method outputs the CAD model with its CAD parameters having their values optimized. The method may comprise a further step of storing the CAD model, for example as a CAD file, and/or inferring (through any known method) a CAM file from the CAD model/CAD file, and for example storing the CAM file, the CAM file serving as control instructions for setting and/or performing the manufacturing process to manufacture the composite part. The method may comprise transmitting such control instructions to a factory, and/or performing the manufacturing process, in which latter case the method is for designing and manufacturing the optimized composite part. Alternatively, the method may be included in such a manufacturing process, but may not comprise the manufacturing step, although the manufacturing is an implied use of the output of the method.

Use cases of the method are now discussed. These use cases illustrate the above-discussed advantages of the proposed method.

**First use case: L-Shape bracket optimization**

This use case notably illustrates the importance of simultaneously considering both shape and anisotropic material properties in a coupled optimization formulation.

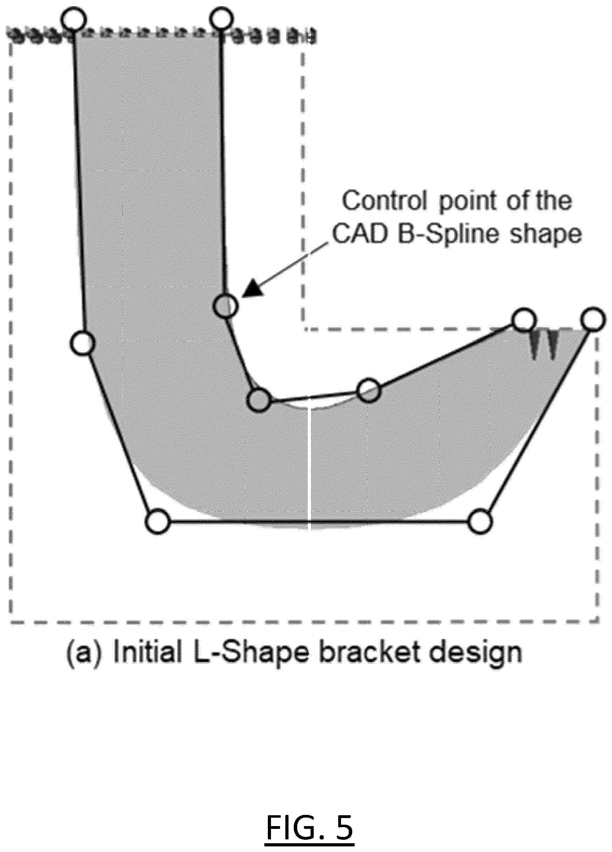

The provided CAD model in this use case represents the L-Shape bracket shown on FIG. 5. The bracket is fixed at the top left and loaded downwards the middle right. The CAD model comprises 20 CAD parameters that controls the shape of the bracket and which consists in 10 sketch control points in 2D. The bracket is made of an isotropic core wrapped by a layer of surface-conformal anisotropic fibers. The one or more material anisotropic property here consists in a density property of the foam core and an angle property which is the orientation of the fibers, which depends on the shape of the bracket and is thus influenced by the CAD parameters that control the shape. The one or more CAD components here consist in one component for the foam core and one component for the conformal fibered layer of constant thickness surrounding the foam core. The one or more use and/or manufacturing performance indicators consist in the stiffness, which is a use performance indicator and is to be maximized, and the mass, which is a use performance indicator and is to be minimized. The optimization program comprises an objective function that specifies these two indicators in that the objective function formulates a weighting between maximizing stiffness and minimizing mass. The anisotropic material fiber is 25 times stiffer in the fiber direction than the other directions of the anisotropic material or compared to the stiffness of the isotropic core. Optimizing the material orientation through optimization of the control points thus allows optimization of the stiffness. Note that the fibers orientations are constrained in the plane of this 2D example. In addition, the fibers are conformal to the surface of the object, which means their orientations are entirely determined by the shape of the outer contour of the object.

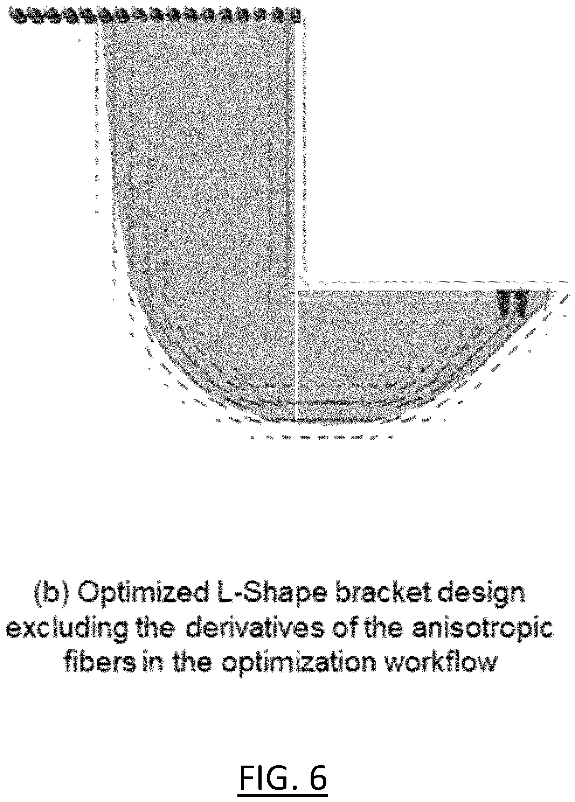

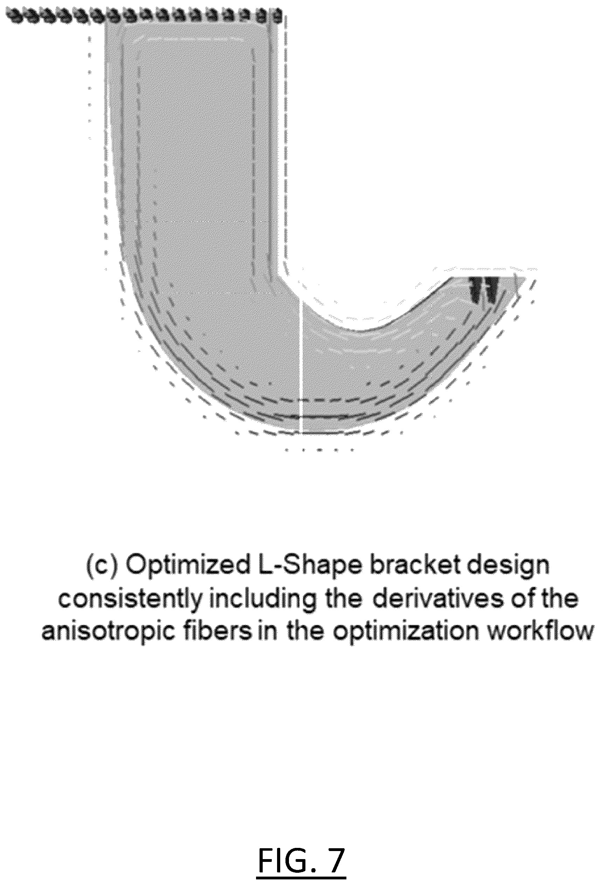

FIG. 6 show the optimized model using an optimization method other than the method of the present disclosure, this other method ignoring the derivatives of the material anisotropy in the chain rule. FIG. 7 shows the optimized model using the method of the proposed disclosure, with the optimization thus including the derivatives of the material anisotropy yielding consistent derivatives. Apart from this difference in use of the derivatives, both optimization setups are identical. In the figures, the short segments overlaid on the surface of the object illustrate the fiber orientations (the grey shades are set directly from the XYZ components of the orientation vector).

It is apparent from the figures that the optimization result, when ignoring the derivatives of the material anisotropy in the chain rule in the optimization, creates a sharp concave corner against the border of the design domain, which is a typical optimization result when optimizing for stiffness using strictly isotropic materials. However, this design is far from optimal for anisotropic materials because the sharp concave corner thereby created by the conformal fibers strongly decreases their load-carrying capacity (concavity concentrates the stress while convexity distributes the stress, thus convex corners are not a problem). The optimization result obtained by the method shows that the consistent derivatives of the anisotropy capture the non-optimality of the sharp concave corners, and therefore, directs the optimization to produce an optimized CAD model having much smoother and continuous fiber orientations around the concave corner. In other words, a new and improved bracket design with increased load-carrying capacity is obtained by using the claimed method, which thus allows fabrication of such a new and improved part based on the CAD model outputted by the method.

The claimed method can guarantee that the optimized part consists of a foam core surrounded by a layer of fibered material with constant thickness and globally conformal angle tangent to the part surface. In an industrial context, this type of design is therefore typically fabricated by CNC (Computer Numerical Control) machining one or several foam blocs according to the shape of the foam core component. Then gluing a sheet of fibered material (such as glass fiber or carbon fiber) around the foam core to obtain lightweight, strong and cheaply produced object. The method may comprise storing the optimized method as a file (*e.g.* a CAM file) of control instructions for performing such manufacturing process and/or performing this machining process that consists in CNC machining and then gluing. It is to be noted that the use case is discussed in 2D for the sake of explanation, but in reality, the bracket has a thickness and is a 3D object, and the manufacturing is in 3D.

This comparative example highlights the advantage of the present approach as it allows mathematically and consistently to include the non-trivial material interactions of the physical model and trace these interactions back to the CAD parameters. In this case, the CAD parameters influence the shape of the outer profile, which influences the orientations and curvatures of the conformal laminate sheet. These CAD parameters then influences the local fiber orientations, which finally affects the load carrying capacity and the overall mechanical performance of the object.

**Second use case: 3D wind turbine blade optimization**

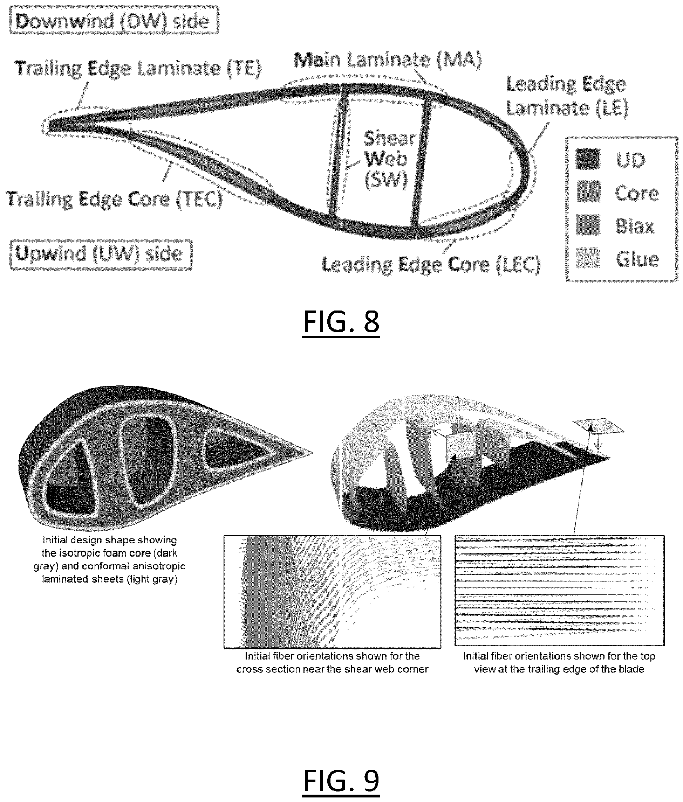

The second use case concerns optimization of a 3D wind turbine blade optimization. FIG. 8 shows a cross-section of the 3D wind turbine blade. The CAD model of the 3D wind turbine is defined by an outer profile having an external prescribed shape obtained from fluid dynamics consideration, and an internal stiffening structure which will be the subject of the optimization considering multiple load cases (torsion and bending). As illustrated in FIG. 8, the material forming the blade consists of composite sandwich structures having a soft lightweight foam core in the middle of the sandwich between the two outer surface anisotropic glass-fiber sheets. It is worth noting that practical industrial models would have stacks of many different oriented plies for each sheet. However, for visualizing, the present optimization result has one fiber orientation per sheet. This is achieved without loss of generality since the method can capture the homogenized properties of one or many oriented plies for the anisotropic constitutive law of each element. The initial design (*i.e.* the CAD model with the initial values of the CAD parameters) is shown in FIG. 9, and has anisotropic and isotropic regions. The fiber orientations are visualized as short segments for the anisotropic regions (the shades of grey are shown as the XYZ components of the orientation vector).

The CAD parameters to optimize are as follows. First, one set of CAD parameters sets the fiber orientation of the outer sheet, this set of CAD parameters consisting in 4 angles to optimize. In addition, the shapes of the 3 internal sheets are controlled by a collection of 22 geometrical CAD parameters. These chosen 26 CAD parameters allow the optimization of the foam core thickness, shear web shapes, and fiber orientations throughout the model, which form the one or more anisotropic material properties. Note that this type of composite structure is manufactured by placing foam blocks of varying thicknesses then unrolling and gluing the laminated sheets over the surface. Consequently, the sheet shapes must be developable surfaces (i.e. zero Gaussian curvatures) and the fiber orientations must be globally conformal to the surfaces in order to be manufacturable for this manufacturing approach. This type of geometrical manufacturing restriction is extremely challenging to enforce in topology optimization but is comparatively easy when optimizing using a CAD representation as enabled by the present method. The one or more CAD components here consist in one component for the foam core, one component for the conformal composite sheet on the outer surface of the part, and three components for the three composite sheets on the inner surfaces of the part.

The performance indicators include two use performance indicators, the mass (to be minimized) and the compliance (to be minimized as well), which is the inverse of the stiffness. These use performance indicators may be captured in the optimization program by an objective function that aims at minimizing both the mass and the compliance.

The CAD parameters of the model may further include CAD parameters that constraint the model to be manufacturable by enforcing the following constraint: the developability of the surfaces formed by the sheet shapes, and the fact that the fiber orientations must be globally conformal to the surfaces. These CAD parameters may consist in a sequence of known CAD operations (*e.g.* sketches, extrudes, fillets). As explained above, these constraints on the CAD parameters of the CAD model allow manufacturability of the blade by the above-discussed manufacturing approach. This illustrates the above-discussed advantage that the CAD parameters may be optimized while being constrained to enforce manufacturability of the composite part. In other words, while formulating, for example, the conformality constraint in terms of KPIs is infeasible in practice, the parametrization by CAD parameters is easy and allows to enforce these constraints. This illustrates an important advantage of directly optimizing the CAD parameters as explained above: manufacturability is guaranteed.

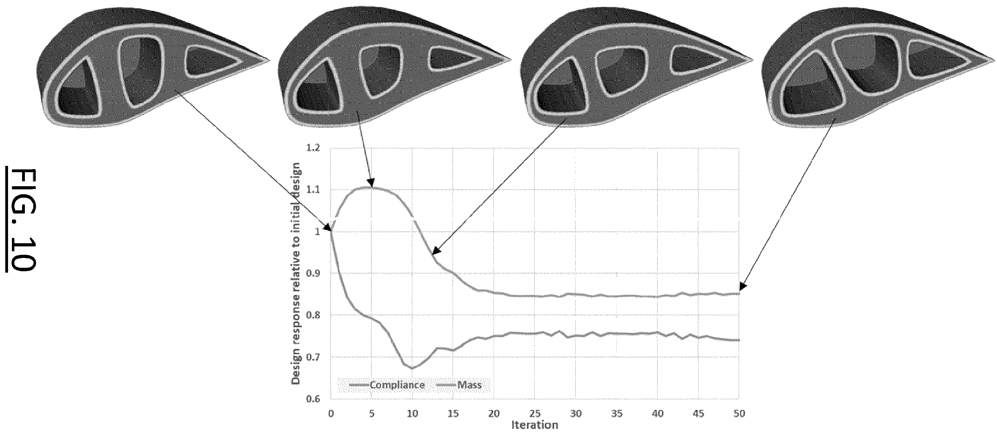

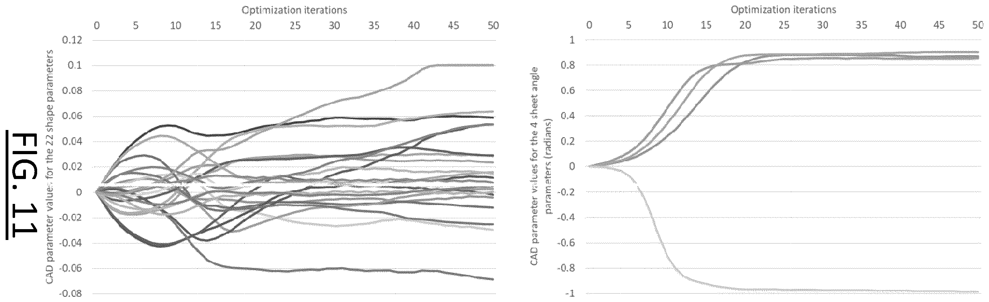

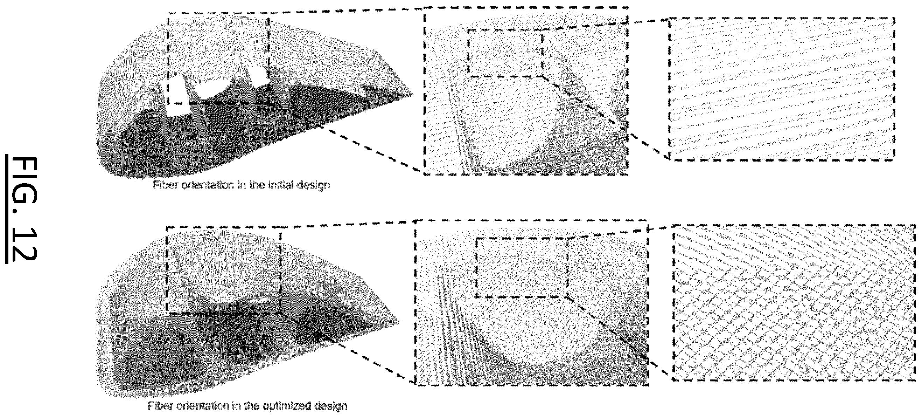

The convergence history of the optimization shows how the optimization smoothly modifies the internal structure of the wing turbine blade to reduce both the mass and compliance of the design compared to the mechanical performance of the design having the initial CAD parameter values. This is illustrated by FIG. 10 and FIG. 11. As illustrated on FIG. 12, the fiber orientations of the optimized model compared to the initial design show that the design changed from a parallel configuration for the fibers to an orthogonal orientation having fiber angles at 90 degrees to each other in the composite sandwich structure. Additionally, it can be also seen on FIG. 12 that the fiber orientations did remain globally consistent with the restriction of being conformal to optimized surfaces.

The output of the method is thus the CAD model of the blade with the above-discussed 4 angles and 22 geometrical CAD parameters optimized to minimize the mass and the compliance of the blade while respecting constraints enforcing the sheet shapes to be developable surfaces and the fiber orientations to be globally conformal to the surfaces. This optimized design is efficiently achieved by the method and is manufacturable by a manufacturing process that consists in placing foam blocks of varying thicknesses then unrolling and gluing the laminated sheets over the surfaces. Such a process may directly be set/controlled according to the optimized values of the CAD parameters. The manufactured part exhibits improved performances with respect to minimal mass and minimal compliance.

**Third use case: wood frame optimization**

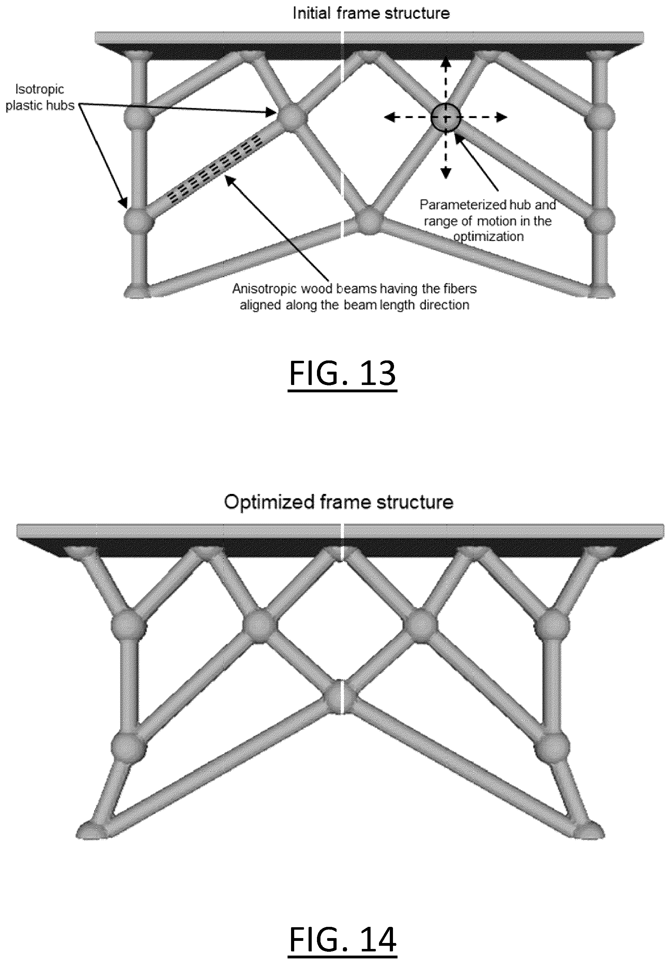

The composite part is here a wood frame supporting the downward load of a top plate. The frame is modeled by a CAD model using 18 straight wood beams having the fibers aligned along the length directions of the beams. These beams are connected at spherical hubs made of isotropic plastic. The CAD parameters consist in the positions of the hubs. Thus, the optimization controls the positions of these hubs which therefore indirectly influences the total lengths of the beams and the fiber orientations throughout the design space. The total lengths of the beams and the fiber orientations form the one or more anisotropic material properties. The one or more CAD components here consist in one component for each wood beam and one component for each isotropic plastic hub. The optimization program optimizes a use performance indicator which consists of the response to the load of the top plate. Specifically, the three KPI consist in minimizing the mass, minimizing the top plate global absolute displacement (the top plate must remain in the same location) and minimizing the top plate local displacements relative to its rest shape (the top plate must retain its original shape). FIG. 13 shows the initial design and FIG. 14 shows the optimized frame structure.

Note, the previously mentioned moving morphable component (MMC) method, if used for performing this optimization, has the drawback that these MMC approaches have issues handling the optimization of the "hubs". The members connected at the hubs often overlap and the hub is geometrically not well-defined because MMC does not properly capture the interaction between the components. In contrast, the CAD parameterization allows enforcing a globally consistent and coherent arrangement of the geometrical features.

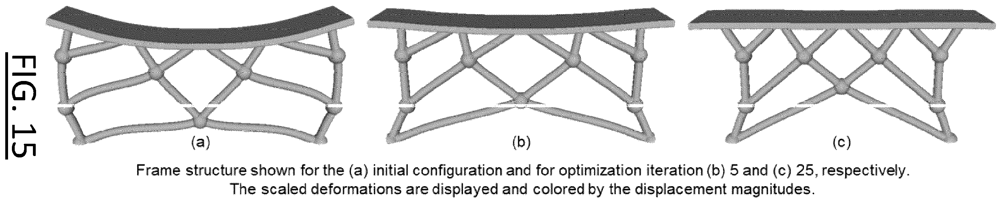

FIG. 15 shows the deformation for the (a) initial configuration and for optimization iteration (b) 5 and (c) 25, respectively, using the proposed method. It can be seen on the figure that the optimized structure is not only stiffer but also carries the top plate more evenly.

The optimized wooden frame structure may be fabricated by cutting several wooden beams to the length of each beam component, and fabricating the plastic hubs (e.g. via plastic injection molding). Then assembling the whole structure by inserting the beams in their hubs and finally securing the assembly with fasteners. The method may comprise such a manufacturing process, and/or storing a file storing the optimized model as manufacturing instructions and/or transmitting the file to the factory performing the manufacturing.

The method generally manipulates modeled objects, such as the CAD model. A modeled object is any object defined by data stored e.g. in the database. By extension, the expression "modeled object" designates the data itself. According to the type of the system, the modeled objects may be defined by different kinds of data. The system may indeed be any combination of a CAD system, a CAE system, a CAM system, a PDM system and/or a PLM system. In those different systems, modeled objects are defined by corresponding data. One may accordingly speak of CAD object, PLM object, PDM object, CAE object, CAM object, CAD data, PLM data, PDM data, CAM data, CAE data. However, these systems are not exclusive one of the other, as a modeled object may be defined by data corresponding to any combination of these systems. A system may thus well be both a CAD, CAE, PLM and/or CAM system, as will be apparent from the definitions of such systems provided below.

By CAD solution (*e.g.* a CAD system or a CAD software), it is additionally meant any system, software or hardware, adapted at least for designing a modeled object on the basis of a graphical representation of the modeled object and/or on a structured representation thereof (*e.g.* a feature tree), such as CATIA. In this case, the data defining a modeled object comprise data allowing the representation of the modeled object. A CAD system may for example provide a representation of CAD modeled objects using edges or lines, in certain cases with faces or surfaces. Lines, edges, or surfaces may be represented in various manners, e.g. non-uniform rational B-splines (NURBS). Specifically, a CAD file contains specifications, from which geometry may be generated, which in turn allows for a representation to be generated. Specifications of a modeled object may be stored in a single CAD file or multiple ones. The typical size of a file representing a modeled object in a CAD system is in the range of one Megabyte per part. And a modeled object may typically be an assembly of thousands of parts.

In the context of CAD, a modeled object may typically be a 2D or 3D modeled object, e.g. representing a product such as a part or an assembly of parts, or possibly an assembly of products. The 2D or 3D modeled object may be a manufacturing product, *i.e.* a product to be manufactured. By "3D modeled object", it is meant any object which is modeled by data allowing its 3D representation. A 3D representation allows the viewing of the part from all angles. For example, a 3D modeled object, when 3D represented, may be handled and turned around any of its axes, or around any axis in the screen on which the representation is displayed. This notably excludes 2D icons, which are not 3D modeled. The display of a 3D representation facilitates design (i.e. increases the speed at which designers statistically accomplish their task). This speeds up the manufacturing process in the industry, as the design of the products is part of the manufacturing process.

The 2D or 3D modeled object represents the geometry of a product to be manufactured in the real world subsequent to the completion of its virtual design with for instance a CAD/CAE software solution or CAD/CAE system, such as a (e.g. mechanical) part or assembly of parts (or equivalently an assembly of parts, as the assembly of parts may be seen as a part itself from the point of view of the method, or the method may be applied independently to each part of the assembly), or more generally any rigid body assembly (e.g. a mobile mechanism). A CAD/CAE software solution allows the design of products in various and unlimited industrial fields, including: aerospace, architecture, construction, consumer goods, high-tech devices, industrial equipment, transportation, marine, and/or offshore oil/gas production or transportation. The 3D modeled object designed by the method may thus represent an industrial product which may be any mechanical part, such as a part of a terrestrial vehicle (including e.g. car and light truck equipment, racing cars, motorcycles, truck and motor equipment, trucks and buses, trains), a part of an aerial vehicle (including e.g. airframe equipment, aerospace equipment, propulsion equipment, defense products, airline equipment, space equipment), a part of a naval vehicle (including e.g. navy equipment, commercial ships, offshore equipment, yachts and workboats, marine equipment), a general mechanical part (including e.g. industrial manufacturing machinery, heavy mobile machinery or equipment, installed equipment, industrial equipment product, fabricated metal product, tire manufacturing product), an electro-mechanical or electronic part (including e.g. consumer electronics, security and/or control and/or instrumentation products, computing and communication equipment, semiconductors, medical devices and equipment), a consumer good (including e.g. furniture, home and garden products, leisure goods, fashion products, hard goods retailers' products, soft goods retailers' products), a packaging (including e.g. food and beverage and tobacco, beauty and personal care, household product packaging).

A CAD system may be history-based. In this case, a modeled object is further defined by data comprising a history of geometrical features. A modeled object may indeed be designed by a physical person (i.e. the designer/user) using standard modeling features (e.g. extrude, revolute, cut, and/or round) and/or standard surfacing features (e.g. sweep, blend, loft, fill, deform, and/or smoothing). Many CAD systems supporting such modeling functions are history-based system. This means that the creation history of design features is typically saved through an acyclic data flow linking the said geometrical features together through input and output links. The history-based modeling paradigm is well known since the beginning of the 80's. A modeled object is described by two persistent data representations: history and B-rep (i.e. boundary representation). The B-rep is the result of the computations defined in the history. The shape of the part displayed on the screen of the computer when the modeled object is represented is (e.g. a tessellation of) the B-rep. The history of the part is the design intent. Basically, the history gathers the information on the operations which the modeled object has undergone. The B-rep may be saved together with the history, to make it easier to display complex parts. The history may be saved together with the B-rep in order to allow design changes of the part according to the design intent.

By PLM system, it is additionally meant any system adapted for the management of a modeled object representing a physical manufactured product (or product to be manufactured). In a PLM system, a modeled object is thus defined by data suitable for the manufacturing of a physical object. These may typically be dimension values and/or tolerance values. For a correct manufacturing of an object, it is indeed better to have such values.

By CAE solution, it is additionally meant any solution, software of hardware, adapted for the analysis of the physical behavior of a modeled object. A well-known and widely used CAE technique is the Finite Element Model (FEM) which is equivalently referred to as CAE model hereinafter. An FEM typically involves a division of a modeled object into elements, i.e., a finite element mesh, which physical behaviors can be computed and simulated through equations. Such CAE solutions are provided by Dassault Systèmes under the trademark SIMULIA®. Another growing CAE technique involves the modeling and analysis of complex systems composed a plurality of components from different fields of physics without CAD geometry data. CAE solutions allow the simulation and thus the optimization, the improvement and the validation of products to manufacture. Such CAE solutions are provided by Dassault Systèmes under the trademark DYMOLA®. CAE may be used to ensure that various structural requirements (such as, but not limited to, mass, stiffness, strength, durability) are achieved by a new CAD model. Some of these requirements may be called Key Performance Indicators (KPIs). For many industrial products (for example cars, airplanes, consumer packaged goods, hi-tech), these KPIs are in conflict e.g. lower mass usually causes lower stiffness. Thus, optimization methods are often applied to find the best trade-off between the KPIs.

By CAM solution, it is meant any solution, software of hardware, adapted for managing the manufacturing data of a product. The manufacturing data generally include data related to the product to manufacture, the manufacturing process and the required resources. A CAM solution is used to plan and optimize the whole manufacturing process of a product. For instance, it may provide the CAM users with information on the feasibility, the duration of a manufacturing process or the number of resources, such as specific robots, that may be used at a specific step of the manufacturing process; and thus allowing decision on management or required investment. CAM is a subsequent process after a CAD process and potential CAE process. For example, a CAM solution may provide the information regarding machining parameters, or molding parameters coherent with a provided extrusion feature in a CAD model. Such CAM solutions are provided by Dassault Systèmes under the trademarks CATIA, Solidworks or trademark DELMIA®.

CAD and CAM solutions are therefore tightly related. Indeed, a CAD solution focuses on the design of a product or part and CAM solution focuses on how to make it. Designing a CAD model is a first step towards a computer-aided manufacturing. Indeed, CAD solutions provide key functionalities, such as feature based modeling and boundary representation (B-Rep), to reduce the risk of errors and the loss of precision during the manufacturing process handled with a CAM solution. Indeed, a CAD model is intended to be manufactured. Therefore, it is a virtual twin, also called digital twin, of an object to be manufactured with two objectives:

- checking the correct behavior of the object to be manufactured in a specific environment; and
- ensuring the manufacturability of the object to be manufactured.

PDM stands for Product Data Management. By PDM solution, it is meant any solution, software of hardware, adapted for managing all types of data related to a particular product. A PDM solution may be used by all actors involved in the lifecycle of a product: primarily engineers but also including project managers, finance people, sales people and buyers. A PDM solution is generally based on a product-oriented database. It allows the actors to share consistent data on their products and therefore prevents actors from using divergent data. Such PDM solutions are provided by Dassault Systèmes under the trademark ENOVIA®.

The modeled object optimized by the method is a CAD model, for example comprising or consisting in a feature tree and/or a B-rep. Such a model may stem from a CAE model and may results from a CAE to CAD conversion process, that the method may for example comprise at an initial stage.

The CAD model is feature-based (*e.g.* it comprises a feature tree, and optionally a corresponding B-rep obtained by executing the feature tree). A feature-based 3D model allows (*e.g.* during the determination of a manufacturing file or CAM file as discussed hereinafter) the detection and an automatic resolution of a geometry error in a CAD model such as a clash that will affect the manufacturing process. A clash is an interpenetration between two parts of a 3D model for example due to their relative motion. Furthermore, this clash may sometimes only be detected via a finite element analysis based on the CAD feature-based model. Therefore, a resolution of a clash can be performed with or automatically by the CAD solution by iteratively modifying the parameters of the features and doing a finite element analysis.

As another example, a feature-based 3D model allows (*e.g.* during the determination of a manufacturing file or CAM file as discussed hereinafter) an automatic creation of a toolpath for a machine via a computer numerical control (CNC). With CNC, each object to be manufactured gets a custom computer program, stored in and executed by the machine control unit, a microcomputer attached to the machine. The program contains the instructions and parameters the machine tool will follow. Mills, lathes, routers, grinders and lasers are examples of common machine tools whose operations can be automated with CNC.

A key characteristic of a CAD model is that it may be designed exactly and unambiguously by chaining a small number of high-level parameterized design operations (including for example, but to limited to, sketch, extrusion, chamfer) and edited by modifying its high-level parameters. That this is a key distinction with the polyhedral representations such as a triangular surface mesh which may represent any 3D shape but do not provide modification or parameterization capabilities required in an industrial design context.

As CAD model is a parameterized model of a part/product, it is lighter in terms of memory footprint than other models such as a CAE model. Indeed, instead of storing a collection of discrete geometrical elements such as finite elements, a CAD model allows the storing of a list of features and parameters, which is lighter in terms of storage and memory footprint. Working on CAD models thus reduced memory requirements for the underlying systems, as compared for example to CAE models, in addition to facilitate editability of the model. This amounts to say that a CAE to CAD conversion process in fact compresses the CAE model into a CAD model which is lighter in terms of memory requirements (e.g. footprint), in addition to transforming the CAE model into a more easily editable CAD model.

The generation of a custom computer program from CAD files may be automated. Such generation may therefore be errorproof and may ensure a perfect reproduction of the CAD model to a manufactured product. CNC is considered to provide more precision, complexity and repeatability than is possible with manual machining. Other benefits include greater accuracy, speed and flexibility, as well as capabilities such as contour machining, which allows milling of contoured shapes, including those produced in 3D designs.

The B-rep (i.e. boundary representation) is a 3D representation of a mechanical part. Specifically, the B-rep is a persistent data representation describing the 3D modeled object representing the mechanical part. The B-rep may be the result of computations and/or a series of operations carried out during a designing phase of the 3D modeled object representing the mechanical part. The shape of the mechanical part displayed on the screen of the computer when the modeled object is represented is (e.g. a tessellation of) the B-rep. In examples, the B-rep represents a part of the model object.

A B-Rep includes topological entities and geometrical entities. Topological entities are: face, edge, and vertex. Geometrical entities are 3D objects: surface, plane, curve, line, point. By definition, a face is a bounded portion of a surface, named the supporting surface. An edge is a bounded portion of a curve, named the supporting curve. A vertex is a point in 3D space. They are related to each other as follows. The bounded portion of a curve is defined by two points (the vertices) lying on the curve. The bounded portion of a surface is defined by its boundary, this boundary being a set of edges lying on the surface. The boundary of the edges of the face are connected by sharing vertices. Faces are connected by sharing edges. Two faces are adjacent if they share an edge. Similarly, two edges are adjacent if they share a vertex. In the CAD system, the B-Rep gathers in an appropriate data structure the "is bounded by" relationship, the relationship between topological entities and supporting geometries, and mathematical descriptions of supporting geometries. An internal edge of a B-Rep is an edge shared by exactly two faces. By definition, a boundary edge is not shared, it bounds only one face. By definition, a boundary face is bounded by at least one boundary edge. A B-Rep is said to be closed if all its edges are internal edges. A B-Rep is said to be open is it includes at least one boundary edge. A closed B-Rep is used to model a thick 3D volume because it defines the inside portion of space (virtually) enclosing material. An open B-Rep is used to model a 3D skin, which represents a 3D object the thickness of which is sufficiently small to be ignored.

A key advantage of the B-Rep over any other representation types used in CAD modeling is its ability to represent arbitrary shapes exactly. All other representations in use, such as point clouds, distance fields and meshes, perform an approximation of the shape to represent by discretization. The B-Rep, on the other hand, contains surface equations that represent the exact design and therefore constitutes a true "master model" for further manufacturing, whether this be generation of toolpaths for CNC, or discretizing into the correct sample density for a given 3D Printer technology. In other words, by using a B-Rep, the 3D model may be an exact representation of the manufactured object. The B-Rep is also advantageous for simulating the behavior of a 3D model. In terms of stress, thermal, electromagnetic or other analysis, it supports local refinement of the simulation meshes to capture physical phenomena, and for kinematics it supports true contact modeling between curved surfaces. Finally, a B-Rep allows a small memory and/or file footprint. First, because the representation contains surfaces based only on parameters. In other representations such as meshes, the equivalent surface comprises up to thousands of triangles. Second, because a B-Rep doesn't contain any history-based information.

The method may be included in a production/manufacturing process, which may comprise, after performing the method, producing a physical product corresponding to the modeled object (CAD model) optimized by the method. The production process may comprise the following steps:

- (*e.g.* automatically) applying the method, thereby obtaining the optimized CAD model outputted by the method;
- using the obtained CAD model for manufacturing the part/product.

Using a CAD model for manufacturing designates any real-world action or series of action that is/are involved in/participate to the manufacturing of the part represented by the CAD model. Using the CAD model for manufacturing may for example comprise one or more of the following steps:

- editing the obtained optimized CAD model;
- performing simulation(s) based on the CAD model or on a corresponding CAE model (*e.g.* the CAE model from which the CAD model stems, after a CAE to CAD conversion process), such as simulations for validation of mechanical, use and/or manufacturing properties and/or constraints (*e.g.* structural simulations, thermodynamics simulation, aerodynamic simulations);
- editing the CAD model based on the results of the simulation(s);
- optionally (*i.e.* depending on the manufacturing process used, the production of the mechanical product may or may not comprise this step), (*e.g.* automatically) determining a manufacturing file/CAM file (*e.g.* comprising manufacturing instructions for manufacturing the product represented by the CAD model and/or control instructions for the manufacturing process and/or for commanding the manufacturing process or manufacturing tool(s) thereof) based on the (*e.g.* edited) CAD model (*e.g.* the control instructions stem from the CAD file storing the CAD model and/or specifications of the CAD model), for production/manufacturing of the manufacturing product;
- sending the CAD file and/or the manufacturing file/CAM file to a factory in view of manufacturing the product represented by the CAD model; and/or
- (*e.g.* automatically) producing/manufacturing, based on the determined manufacturing file/CAM file or on the CAD model, the mechanical product originally represented by the model outputted by the method. This may include feeding (*e.g.* automatically) the manufacturing file/CAM file and/or the CAD file to the machine(s) performing the manufacturing process.

This last step of production/manufacturing may be referred to as the manufacturing step or production step. This step manufactures/fabricates the part/product based on the CAD model and/or the CAM file, e.g. upon the CAD model and/or CAD file being fed to one or more manufacturing machine(s) or computer system(s) controlling the machine(s). The manufacturing step may comprise performing any known manufacturing process or series of manufacturing processes, for example one or more additive manufacturing steps, one or more cutting steps (e.g. laser cutting or plasma cutting steps), one or more stamping steps, one or more forging steps, one or more bending steps, one or more deep drawing steps, one or more molding steps, one or more machining steps (e.g. milling steps) and/or one or more punching steps. Because the design method improves the design of a model (CAE or CAD) representing the part/product, the manufacturing and its productivity are also improved.

Editing the CAD model may comprise, by a user (*i.e.* a designer), performing one or more editions of the CAD model, e.g. by using a CAD solution. The modifications of the CAD model may include one or more modifications each of a geometry and/or of a parameter of the CAD model. The modifications may include any modification or series of modifications performed on a feature tree of the model (*e.g.* modification of feature parameters and/or specifications) and/or modifications performed on a displayed representation of the CAD model (*e.g.* a B-rep). The modifications are modifications which maintain the technical functionalities of the part/product, *i.e.* the user performs modifications which may affect the geometry and/or parameters of the model but only with the purpose of making the CAD model technically more compliant with the downstream use and/or manufacturing of the part/product. Such modifications may include any modification or series of modification that make the CAD model technically compliant with specifications of the machine(s) used in the downstream manufacturing process. Such modifications may additionally or alternatively include any modification or series of modification that make the CAD model technically compliant with a further use of the product/part once manufactured, such modification or series of modifications being for example based on results of the simulation(s).

The CAM file may comprise a manufacturing set up model obtained from the CAD model. The manufacturing set up may comprise all data required for manufacturing the mechanical product so that it has a geometry and/or a distribution of material that corresponds to what is captured by the CAD model, possibly up to manufacturing tolerance errors. Determining the production file may comprise applying any CAM (Computer-Aided Manufacturing) or CAD-to-CAM solution for (*e.g.* automatically) determining a production file from the CAD model (*e.g.* any automated CAD-to-CAM conversion algorithm). Such CAM or CAD-to-CAM solutions may include one or more of the following software solutions, which enable automatic generation of manufacturing instructions and tool paths for a given manufacturing process based on a CAD model of the product to manufacture:

- Fusion 360,
- FreeCAD,
- CATIA,
- SOLIDWORKS,
- The NC Shop Floor programmer of Dassault Systèmes illustrated on https://my.3dexperience.3ds.com/welcome/fr/compass-world/rootroles/nc-shop-floor-programmer,
- The NC Mill-Turn Machine Programmer of Dassault Systèmes illustrated on https://my.3dexperience.3ds.com/welcome/fr/compass-world/rootroles/nc-mill-turn-machine-programmer, and/or
- The Powder Bed Machine Programmer of Dassault Systèmes illustrated on https://my.3dexperience.3ds.com/welcome/fr/compass-world/rootroles/powder-bed-machine-programmer.

The product/part may be an additive manufacturable part, *i.e.* a part to be manufactured by additive manufacturing (*i.e.* 3D printing). In this case, the production process does not comprise the step of determining the CAM file and directly proceeds to the producing/manufacturing step, by directly (*e.g.* and automatically) feeding a 3D printer with the CAD model. 3D printers are configured for, upon being fed with a CAD model representing a mechanical product (*e.g.* and upon launching, by a 3D printer operator, the 3D printing), directly and automatically 3D print the mechanical product in accordance with the CAD model. In other words, the 3D printer receive the CAD model, which is (*e.g.* automatically) fed to it, reads (*e.g.* automatically) the CAD model, and prints (*e.g.* automatically) the part by adding together material, *e.g.* layer by layer, to reproduce the geometry and/or distribution of material captured by the CAD model. The 3D printer adds the material to thereby reproduce exactly in reality the geometry and/or distribution of material captured by the CAD model, up to the resolution of the 3D printer, and optionally with or without tolerance errors and/or manufacturing corrections. The manufacturing may comprise, *e.g.* by a user (*e.g.* an operator of the 3D printer) or automatically (by the 3D printer or a computer system controlling it), determining such manufacturing corrections and/or tolerance errors, for example by modifying the CAD file to match specifications of the 3D printer. The production process may additionally or alternatively comprise determining (*e.g.* automatically by the 3D printer or a computer system controlling it) from the CAD model, a printing direction, for example to minimize overhang volume (as described in European patent No. 3327593, which is incorporated herein by reference), a layer-slicing (i.e., determining thickness of each layer, and layer-wise paths/trajectories and other characteristics for the 3D printer head (e.g., for a laser beam, for example the path, speed, intensity/temperature, and other parameters).

The product/part may alternatively be a machined part (*i.e.* a part manufactured by machining), such as a milled part (*i.e.* a part manufactured by milling). In such a case, the production process may comprise a step of determining the CAM file. This step may be carried out automatically, by any suitable CAM solution to automatically obtain a CAM file from a CAD model of a machined part. The determination of the CAM file may comprise (*e.g.* automatically) checking if the CAD model has any geometric particularity (*e.g.* error or artefact) that may affect the production process and (*e.g.* automatically) correcting such particularities. For example, machining or milling based on the CAD model may not be carried out if the CAD model still comprises sharp edges (because the machining or milling tool cannot create sharp edges), and in such a case the determination of the CAM file may comprise (*e.g.* automatically) rounding or filleting such sharp edges (*e.g.* with a round or fillet radius that corresponds, *e.g.* substantially equals up to a tolerance error, the radius of the cutting head of the machining tool), so that machining or milling based on the CAD model can be done. More generally, the determination of the CAM file may automatically comprise rounding or filleting geometries within the CAD model that are incompatible with the radius of the machining or milling tool, to enable machining/milling. This check and possible corrections (*e.g.* rounding or filleting of geometries) may be carried out automatically as previously discussed, but also, by a user (*e.g.* a machining engineer), which performs the correction by hand on a CAD and/or CAM solution, *e.g.* the solution constraining the user to perform corrections that make the CAD model compliant with specifications of the tool used in the machining process.

Further to the check, the determination of the CAM file may comprise (*e.g.* automatically) determining the machining or milling path, *i.e.* the path to be taken by the machining tool to machine the product. The path may comprise a set of coordinates and/or a parameterized trajectory to be followed by the machining tool for machining, and determining the path may comprise (*e.g.* automatically) computing these coordinates and/or trajectory based on the CAD model. This computation may be based on the computation of a boundary of a Minkowski subtraction of the CAD model by a CAD model representation of the machining tool, as for example discussed in European Patent Application EP21306754.9 filed on 13 December 2021 by Dassault Systèmes, and which is incorporated herein by reference. It is to be understood that the path may be a single path, *e.g.* that the tool continuously follows without breaking contact with the material to be cut. Alternatively, the path may be a concatenation of a sequence sub-paths to be followed in a certain order by the tool, *e.g.* each being continuously followed by the tool without breaking contact with the material to be cut. Optionally, the determination of the CAM file may then comprise (*e.g.* automatically) setting machine parameters, including cutting speed, cut/pierce height, and/or mold opening stroke, for example based on the determined path and on the specification of the machine. Optionally, the determination of the CAM file may then comprise (*e.g.* automatically) configuring nesting where the CAM solution decides the best orientation for a part to maximize machining efficiency.

In this case of a machining or milling part, the determining of the CAM file thus results in, and outputs, the CAM file comprising a machining path, and optionally the set machine parameters and/or specifications of the configured nesting. This outputted CAM file may be then (*e.g.* directly and automatically) fed to the machining tool and/or the machining tool may then (*e.g.* directly and automatically) be programmed by reading the file, upon which the production process comprises the producing/manufacturing step where the machine performs the machining of the product according to the production file, *e.g.* by directly and automatically executing the production file. The machining process comprises the machining tool cutting a real-world block of material to reproduce the geometry and/or distribution of material captured by the CAD model, *e.g.* up to a tolerance error (*e.g.* tens of microns for milling).

The product/part may alternatively be a molded part, *i.e.* a part manufactured by molding (*e.g.* injection-molding). In such a case, the production process may comprise the step of determining the CAM file. This step may be carried out automatically, by any suitable CAM solution to automatically obtain a CAM file from a CAD model of a molded part. The determining of the CAM file may comprise (*e.g.* automatically) performing a sequence of molding checks based on the CAD model to check that the geometry and/or distribution of material captured by the CAD model is adapted for molding, and (*e.g.* automatically) performing the appropriate corrections if the CAD model is not adapted for molding. Performing the checks and the appropriate corrections (if any) may be carried out automatically, or, alternatively, by a user (*e.g.* a molding engineer), for example using a CAD and/or CAM solution that allows a user to perform the appropriate corrections on the CAD model but constraints him/her corrections that make the CAD model compliant with specifications of the molding tool(s). The checks may include: verifying that the virtual product as represented by the CAD model is consistent with the dimensions of the mold and/or verifying that the CAD model comprises all the draft angles required for demolding the product, as known *per se* from molding. The determining of the CAM file may then further comprise determining, based on the CAD model, a quantity of liquid material to be used for molding, and/or a time to let the liquid material harden/set inside the mold, and outputting a CAM file comprising these parameters. The production process then comprises (*e.g.* automatically) performing the molding based on the outputted file, where the mold shapes, for the determined hardening time, a liquid material into a shape that corresponds to the geometry and/or distribution of material captured by the CAD model, *e.g.* up to a tolerance error (*e.g.* up to the incorporation of draft angles or to the modification of draft angles, for demolding).

The product/part may alternatively be a stamped part, also possibly referred to as "stamping part", *i.e.* a part to be manufactured in a stamping process. The production process may in this case comprise (*e.g.* automatically) determining a CAM file based on the CAD model. The CAD model represents the stamping part, *e.g.* possible with one or more flanges if the part is to comprise some, and possibly in this latter case with extra material to be removed so as to form an unfolded state of one or more flanges of the part, as known *per se* from stamping. The CAD model thus comprises a portion that represents the part without the flanges (which is the whole part in some cases) and possibly an outer extra patch portion that represents the flanges (if any), with possibly the extra material (if any). This extra patch portion may present a g2-continuity over a certain length and then a g1-continuity over a certain length.

The determination of the CAM file may in this stamping case comprise (*e.g.* automatically) determining parameters of the stamping machine, for example a size of a stamping die or punch and/or a stamping force, based on the geometry and/or distribution of material of the virtual product as captured by the CAD model. If the CAD model also comprises the representation of the extra material to be removed so as to form an unfolded state of one or more flanges of the part, the extra material to be removed may for example be cut by machining, and determining the CAM file may also comprise determining a corresponding machining CAM file, *e.g.* as discussed previously. If there are one or more flanges, determining the CAM file may comprise determining geometrical specifications of the g2-continuity and g1-continuity portions that allow, after the stamping itself and the removal of the extra material, to fold in a folding process the flanges towards an inner surface of the stamped part and along the g2-continuity length. The CAM file thereby determined may thus comprise: parameters of the stamping tool, optionally said specifications for folding the flanges (if any), and optionally a machining production file for removing the extra material (if any).

The stamping production process may then output, *e.g.* directly and automatically, the CAM file, and perform the stamping process (*e.g.* automatically) based on the file. The stamping process may comprise stamping (*e.g.* punching) a portion of material to form the product as represented by the CAD file, that is possibly with the unfolded flanges and the extra material (if any). Where appropriate, the stamping process may then comprise cutting the extra material based on the machining production file and folding the flanges based on said specifications for folding the flanges, thereby folding the flanges on their g2-continuity length and giving a smooth aspect to the outer boundary of the part. In this latter case, the shape of the part once manufactured differ from its virtual counterpart as represented by the CAD model in that the extra material is removed and the flanges are folded, whereas the CAD model represents the part with the extra material and the flanges in an unfolded state.

The method is computer-implemented. This means that steps (or substantially all the steps) of the method are executed by at least one computer, or any system alike. Thus, steps of the method are performed by the computer, possibly fully automatically, or, semi-automatically. In examples, the triggering of at least some of the steps of the method may be performed through user-computer interaction. The level of user-computer interaction required may depend on the level of automatism foreseen and put in balance with the need to implement user's wishes. In examples, this level may be user-defined and/or pre-defined.

A typical example of computer-implementation of a method is to perform the method with a system adapted for this purpose. The system may comprise a processor coupled to a memory and a graphical user interface (GUI), the memory having recorded thereon a computer program comprising instructions for performing the method. The memory may also store a database. The memory is any hardware adapted for such storage, possibly comprising several physical distinct parts (e.g. one for the program, and possibly one for the database).

FIG. 16 shows an example of the system, wherein the system is a client computer system, *e.g.* a workstation of a user.

The client computer of the example comprises a central processing unit (CPU) 1010 connected to an internal communication BUS 1000, a random access memory (RAM) 1070 also connected to the BUS. The client computer is further provided with a graphical processing unit (GPU) 1110 which is associated with a video random access memory 1100 connected to the BUS. Video RAM 1100 is also known in the art as frame buffer. A mass storage device controller 1020 manages accesses to a mass memory device, such as hard drive 1030. Mass memory devices suitable for tangibly embodying computer program instructions and data include all forms of nonvolatile memory, including by way of example semiconductor memory devices, such as EPROM, EEPROM, and flash memory devices; magnetic disks such as internal hard disks and removable disks; magneto-optical disks. Any of the foregoing may be supplemented by, or incorporated in, specially designed ASICs (application-specific integrated circuits). A network adapter 1050 manages accesses to a network 1060. The client computer may also include a haptic device 1090 such as cursor control device, a keyboard or the like. A cursor control device is used in the client computer to permit the user to selectively position a cursor at any desired location on display 1080. In addition, the cursor control device allows the user to select various commands, and input control signals. The cursor control device includes a number of signal generation devices for input control signals to system. Typically, a cursor control device may be a mouse, the button of the mouse being used to generate the signals. Alternatively or additionally, the client computer system may comprise a sensitive pad, and/or a sensitive screen.

The computer program may comprise instructions executable by a computer, the instructions comprising means for causing the above system to perform the method. The program may be recordable on any data storage medium, including the memory of the system. The program may for example be implemented in digital electronic circuitry, or in computer hardware, firmware, software, or in combinations of them. The program may be implemented as an apparatus, for example a product tangibly embodied in a machine-readable storage device for execution by a programmable processor. Method steps may be performed by a programmable processor executing a program of instructions to perform functions of the method by operating on input data and generating output. The processor may thus be programmable and coupled to receive data and instructions from, and to transmit data and instructions to, a data storage system, at least one input device, and at least one output device. The application program may be implemented in a high-level procedural or object-oriented programming language, or in assembly or machine language if desired. In any case, the language may be a compiled or interpreted language. The program may be a full installation program or an update program. Application of the program on the system results in any case in instructions for performing the method. The computer program may alternatively be stored and executed on a server of a cloud computing environment, the server being in communication across a network with one or more clients. In such a case a processing unit executes the instructions comprised by the program, thereby causing the method to be performed on the cloud computing environment.

## Claims

1. A computer-implemented method for optimization of one or more anisotropic material properties of a composite part, the method comprising:

- providing

◦ a Computer-Aided Design (CAD) model representing the composite part, the CAD model including a feature tree having one or more CAD parameters each having an initial value, at least one CAD parameter of the one or more CAD parameters influencing the one or more anisotropic material properties of the composite part; and

◦ an optimization problem specified by one or more use and/or manufacturing performance indicators, the one or more use and/or manufacturing performance indicators comprising one or more objective functions and/or one or more constraints; and

- modifying the initial values of the one or more CAD parameters by solving the optimization problem using a gradient-based optimization method, the optimization method having as free variable the one or more CAD parameters, the optimization method using sensitivities, each sensitivity being an approximation of a respective derivative of a respective performance indicator with respect to a respective CAD parameter of the one or more CAD parameters, the sensitivities being compositions of:

◦ approximated respective derivatives each of a respective performance indicator of the one or more use and/or manufacturing performance indicators with respect to one or more material property fields, each material property field of the one or more material property fields representing a distribution of an anisotropic material property of the one or more anisotropic material properties, and

◦ approximated respective derivatives each of the one or more material property fields with respect to a respective CAD parameter,

the CAD model with the one or more CAD parameters having their initial value modified being stored as a file convertible into control instructions for setting and/or performing the manufacturing process to manufacture the composite part.

2. The method of claim 1, wherein the CAD model is formed by one or more CAD components each corresponding to a region of space, and the method comprising:

- computing one or more signed distance fields each to a respective CAD component of the one or more CAD components; and

- for each given material property field of the one or more material property field and for each element of a mesh of the CAD model, each local material property value of the respective given material property field at this element of the mesh results from a weighted combination of material properties values of the one or more CAD components each weighted by a projection of the signed distance between the element of the mesh and the respective CAD component.

3. The method of claim 2, wherein the projection is a function that maps $]-\infty; +\infty[$ onto $[0; 1]$ and that has well-defined first order derivatives, wherein optionally the function is a smooth Heaviside projection.

4. The method of any one of claims 2 to 3, wherein the weighted combination is a weighted sum.

5. The method of claim 4, wherein

$$M_{ik} = \frac{\sum_{j\in\Omega_{comp}} H_{ij}\, m_{ijk}}{\sum_{j\in\Omega_{comp}} H_{ij}}$$

$\forall\, i \in \Omega_{mesh},\, k \in \Omega_{type}$

where $\Omega_{type}$ is the set of the one or more anisotropic material properties of the composite part, $M_{ik}$ is the local material property value of material property $k$ in $\Omega_{type}$ at element $i$ of the mesh $\Omega_{mesh}$ of the CAD model, $j$ is a component, $\Omega_{comp}$ is the set of all the components, $H_{ij}$ are weights, and $m_{ijk}$ are the material properties of the components.

6. The method of claim 5, wherein each weight $H_{ij}$ is defined by the following smooth Heaviside projection given by the formula

$$H_{ij} = \frac{1}{1 + e^{\frac{SDF_{ij}}{0.2\alpha \times 2l}}},$$

$\forall\, i \in \Omega_{mesh},\, j \in \Omega_{comp}$

where $\alpha \geq 0$ is a steepness coefficient of the smooth Heaviside projection, where $l$ is an average size of the elements in the mesh, and $SDF_{ij}$ is the signed distance value from element i to component j, the signed distance value being a distance from the element i to the outer surface of the component j, the value having a sign when the element corresponds to an inside portion of the component and the opposite sign when the element corresponds to an outside portion of the component.

7. The method of claim 5 or 6, wherein each respective approximated derivative $\frac{\partial M_{ik}}{\partial CAD_p}$ of the one or more material property fields with respect to a respective CAD parameter is of the type:

$$\frac{\partial M_{ik}}{\partial CAD_p} \cong \frac{M_{ik}\left(CAD_p + \frac{h_p}{2}\right) - M_{ik}\left(CAD_p - \frac{h_p}{2}\right)}{h_p},$$

$\forall i \in \Omega_{mesh},\, k \in \Omega_{type},\, p \in \Omega_{param}$

where $CAD_p$ is the respective CAD parameter, where $h_p > 0$ is a small perturbation, where $\Omega_{param}$ is a set of the CAD parameters.

8. The method of claim 7, wherein each sensitivity $\frac{\partial KPI_n}{\partial CAD_p}$ is of the type:

$$\frac{\partial KPI_n}{\partial CAD_p} = \frac{\partial KPI_n}{\partial M_{ik}} \frac{\partial M_{ik}}{\partial CAD_p},$$

$\forall n \in \Omega_{perfo},\, p \in \Omega_{param}$

where $\Omega_{perfo}$ is the set of the one or more use and/or manufacturing performance indicators and $KPI_n$ denotes the value of the use and/or manufacturing performance indicator $n \in \Omega_{perfo}$.

9. The method of any one of claims 2 to 8, wherein the one or more CAD components consist of several components.

10. The method of any one of claims 1 to 9, wherein the one or more anisotropic material properties consist of several anisotropic material properties.

11. The method of any one of claims 1 to 10, wherein the at least one CAD parameter influencing the one or more anisotropic material properties of the composite part satisfies a constraint of a manufacturing process for manufacturing the composite part.

12. A computer program comprising instructions which, when executed by a computer system, cause the computer system to perform the method according to any one of claims 1 to 11.

13. A computer-readable data storage medium having recorded thereon the computer program of claim 12.

14. A computer system comprising a processor coupled to a memory, the memory having recorded thereon the computer program of claim 12.

**Patentansprüche**

1. Computerimplementiertes Verfahren zum Optimieren einer oder mehrerer anisotroper Materialeigenschaften eines Verbundstoffteils, das Verfahren umfassend:

   - Bereitstellen von Folgendem:

     ◦ ein Modell für rechnerunterstütztes Konstruieren (CAD), das das Verbundteil darstellt, wobei das CAD-Modell einen Merkmalsbaum beinhaltet, der einen mehrere CAD-Parameter aufweist, die jeweils einen Anfangswert aufweisen, mindestens einen CAD-Parameter des einen oder der mehreren CAD-Parameter, der die eine oder die mehreren anisotropen Materialeigenschaften des Verbundteils beeinflusst; und
     ◦ ein Optimierungsproblem, das durch einen oder mehrere Verwendungs- und/oder Herstellungsleistungsindikatoren spezifiziert ist, der eine oder die mehreren Verwendungs- und/oder Herstellungsleistungsindikatoren umfassend eine oder mehrere Zielfunktionen und/oder eine oder mehrere Beschränkungen; und

   - Modifizieren der Anfangswerte des einen oder der mehreren CAD-Parameter durch Lösen des Optimierungsproblems unter Verwendung eines gradientenbasierten Optimierungsverfahrens, wobei das Optimierungsverfahren als freie Variable den einen oder die mehreren CAD-Parameter aufweist, wobei das Optimierungsverfahren Sensitivitäten verwendet, wobei jede Sensitivität eine Annäherung an eine jeweilige Ableitung eines jeweiligen Leistungsindikators in Bezug auf einen jeweiligen CAD-Parameter des einen oder der mehreren CAD-Parameter ist, wobei die Sensitivitäten Zusammensetzungen sind von:

     ◦ angenäherte jeweilige Ableitungen eines jeweiligen Leistungsindikators des einen oder der mehreren Verwendungs- und/oder Herstellungsleistungsindikatoren in Bezug auf ein oder mehrere Materialeigenschaftsfelder, wobei jedes Materialeigenschaftsfeld der einen oder der mehreren Materialeigenschaftsfelder eine Verteilung einer anisotropen Materialeigenschaft der einen oder der mehreren anisotropen Materialeigenschaften darstellt, und
     ◦ angenäherte jeweilige Ableitungen jedes der einen oder mehreren Materialeigenschaftsfelder in Bezug auf einen jeweiligen CAD-Parameter,

   wobei das CAD-Modell mit dem einen oder den mehreren CAD-Parametern, deren Anfangswert geändert wurde, als eine Datei gespeichert wird, die in Steueranweisungen für die Einstellung und/oder Durchführung des Herstellungsprozesses zum Herstellen des Verbundstoffteils umgewandelt werden kann.

2. Verfahren nach Anspruch 1, wobei das CAD-Modell durch eine oder mehrere CAD-Komponenten gebildet wird, die jeweils einem Bereich des Raums entsprechen, und das Verfahren Folgendes umfasst:

   - Berechnen eines oder mehrerer vorzeichenbehafteter Abstandsfelder jeweils zu einer entsprechenden CAD-Komponente der einen oder der mehreren CAD-Komponenten; und
   - für jedes gegebene Materialeigenschaftsfeld des einen oder der mehreren Materialeigenschaftsfelder und für jedes Element eines Netzes des CAD-Modells jeder lokale Materialeigenschaftswert des jeweiligen gegebenen Materialeigenschaftsfeldes an diesem Element des Netzes aus einer gewichteten Kombination von Materialeigenschaftswerten der einen oder mehreren CAD-Komponenten, die jeweils durch eine Projektion des vorzeichenbehafteten Abstands zwischen dem Element des Netzes und der jeweiligen CAD-Komponente gewichtet werden, resultiert.

3. Verfahren nach Anspruch 2, wobei die Projektion eine Funktion ist, die ]-∞; +∞[ zu [0; 1] zuordnet und die gut definierten Ableitungen erster Ordnung aufweist, wobei die Funktion optional eine glatte Heaviside-Projektion ist.

4. Verfahren nach einem der Ansprüche 2 bis 3, wobei die gewichtete Kombination eine gewichtete Summe ist.

5. Verfahren nach Anspruch 4, wobei:

$$M_{ik} = \frac{\sum_{j \in \Omega_{comp}} H_{ij}\, m_{ijk}}{\sum_{j \in \Omega_{comp}} H_{ij}}$$

$\forall\ i\ E\ \Omega_{mesh},\ k\ E\ \Omega_{type}$

wobei $\Omega_{type}$ die Menge der einen oder mehreren anisotropen Materialeigenschaften des Verbundbauteils ist, $M_{ik}$ der lokale Materialeigenschaftswert der Materialeigenschaft $k$ in $\Omega_{typ}$ a dem Element $i$ des Netzes $\Omega_{mesh}$ des CAD-Modells ist, $j$ eine Komponente ist, $\Omega_{comp}$ die Menge aller Komponenten ist, $H_{ij}$ Gewichte sind und $m_{ijk}$ die Material-eigenschaften der Komponenten sind.

6. Verfahren nach Anspruch 5, wobei jedes Gewicht $H_{ij}$ durch die folgende glatte Heaviside-Projektion definiert ist, die gegeben ist durch die folgende Formel

$$H_{ij} = \frac{1}{1 + e^{\frac{SDF_{ij}}{0.2\alpha \times 2l}}},$$

$\forall\ i \in \Omega_{mesh},\ j \in \Omega_{comp}$

wobei $\alpha \geq 0$ ein Steilheitskoeffizient der glatten Heaviside-Projektion ist, wobei $l$ eine durchschnittliche Größe der Elemente im Netz ist, und $SDF_{ij}$ der vorzeichenbehaftete Abstandswert von dem Element $i$ zu der Komponente $j$ ist, wobei der vorzeichenbehaftete Abstandswert ein Abstand von dem Element $i$ zu der Außenfläche der Komponente $j$ ist, wobei der Wert ein Vorzeichen, wenn das Element einem inneren Abschnitt der Komponente entspricht, und das entgegengesetzte Vorzeichen, wenn das Element einem äußeren Abschnitt der Komponente entspricht, aufweist.

7. Verfahren nach Anspruch 5 oder 6, wobei jede jeweilige angenäherte Ableitung $\frac{\partial M_{ik}}{\partial CAD_p}$ des einen oder der mehreren Materialeigenschaftsfelder in Bezug auf einen jeweiligen CAD-Parameter von dem folgenden Typ ist:

$$\frac{\partial M_{ik}}{\partial CAD_p} \cong \frac{M_{ik}\left(CAD_p + \frac{h_p}{2}\right) - M_{ik}\left(CAD_p - \frac{h_p}{2}\right)}{h_p},$$

$$\forall i \in \Omega_{mesh},\ k \in \Omega_{type},\ p \in \Omega_{param}$$

wobei $CAD_p$ der jeweilige CAD-Parameter ist, wobei $h_p > 0$ eine kleine Störung ist, wobei $\Omega_{perfo}$ ein Satz von CAD-Parameter ist.

8. Verfahren nach Anspruch 7, wobei jede Sensitivität $\frac{\partial KPI_n}{\partial CAD_p}$ von dem folgenden Typ ist:

$$\frac{\partial KPI_n}{\partial CAD_p} = \frac{\partial KPI_n}{\partial M_{ik}} \frac{\partial M_{ik}}{\partial CAD_p},$$

$\forall n \in \Omega_{perfo},\ p \in \Omega_{param}$

wobei $\Omega_{perfo}$ die Menge des einen oder der mehreren Nutzungs- und/oder Herstellungsleistungsindikatoren ist und $KPI_n$ den Wert des Nutzungs- und/oder Herstellungsleistungsindikators $n \in \Omega_{perfo}$ bezeichnet.

9. Verfahren nach einem der Ansprüche 2 bis 8, wobei die eine oder die mehreren CAD-Komponenten aus mehreren Komponenten bestehen.

10. Verfahren nach einem der Ansprüche 1 bis 9, wobei die eine oder die mehreren anisotropen Materialeigenschaften aus mehreren anisotropen Materialeigenschaften bestehen.

11. Verfahren nach einem der Ansprüche 1 bis 10, wobei der mindestens eine CAD-Parameter, der die eine oder die mehreren anisotropen Materialeigenschaft(en) des Verbundteils beeinflusst, eine Randbedingung eines Herstellungsprozesses zum Herstellen des Verbundteils erfüllt.

**12.** Computerprogramm, umfassend Anweisungen, die, wenn sie von einem Computersystem ausgeführt werden, das Computersystem veranlassen, das Verfahren nach einem der Ansprüche 1 bis 11 durchzuführen.

**13.** Computerlesbares Datenspeichermedium, in dem das Computerprogramm nach Anspruch 12 aufgezeichnet ist.

**14.** Computersystem, umfassend einen Prozessor, der mit einem Speicher gekoppelt ist, wobei in dem Speicher das Computerprogramm nach Anspruch 12 aufgezeichnet ist.

**Revendications**

**1.** Procédé mis en œuvre par ordinateur pour l'optimisation d'une ou plusieurs propriétés de matériau anisotrope d'une pièce composite, le procédé comprenant le fait de :

- fournir :

◦ un modèle de conception assistée par ordinateur (CAD) représentant la pièce composite, le modèle de conception CAD comprenant un arbre de caractéristiques présentant un ou plusieurs paramètres de conception CAD ayant chacun une valeur initiale, au moins un paramètre de conception CAD parmi le ou les paramètres de conception CAD influençant la ou les propriétés de matériau anisotrope de la pièce composite ; et
◦ un problème d'optimisation spécifié par un ou plusieurs indicateurs de performance d'utilisation et/ou de fabrication, le ou les indicateurs de performance d'utilisation et/ou de fabrication comprenant une ou plusieurs fonctions objectif et/ou une ou plusieurs contraintes ; et

- modifier les valeurs initiales du ou des paramètres de conception CAD en résolvant le problème d'optimisation à l'aide d'un procédé d'optimisation basé sur le gradient, le procédé d'optimisation ayant comme variable libre le ou les paramètres de conception CAD, le procédé d'optimisation utilisant des sensibilités, chaque sensibilité étant une approximation d'une dérivée respective d'un indicateur de performance respectif par rapport à un paramètre de conception CAD respectif du ou des paramètres de conception CAD, les sensibilités étant des compositions de :

◦ dérivées respectives approximées, chacune des indicateurs de performance respectifs du ou des indicateurs de performance d'utilisation et/ou de fabrication par rapport à un ou plusieurs champs de propriétés de matériau, chaque champ de propriétés de matériau représentant une distribution d'une propriété de matériau anisotrope de la ou des propriétés de matériau anisotrope, et
◦ dérivées respectives approximées, chacune du ou des champs de propriétés de matériau par rapport à un paramètre de conception CAD respectif,

le modèle de conception CAD avec le ou les paramètres de conception CAD dont la valeur initiale a été modifiée étant stocké sous la forme d'un fichier convertible en instructions de commande pour définir et/ou exécuter le processus de fabrication afin de fabriquer la pièce composite.

**2.** Procédé selon la revendication 1, dans lequel le modèle de conception CAD est formé par un ou plusieurs composants de conception CAD correspondant chacun à une région de l'espace, et le procédé comprenant le fait de :

- calculer un ou plusieurs champs de distance signée, correspondant chacun à un composant de conception CAD respectif parmi le ou les composants de conception CAD ; et
- pour chaque champ de propriétés de matériau donné parmi le ou les champs de propriétés de matériau et pour chaque élément d'un maillage du modèle de conception CAD, chaque valeur de propriétés de matériau locales du champ de propriétés de matériau donné respectif au niveau de cet élément du maillage résulte d'une combinaison pondérée de valeurs de propriétés de matériau du ou des composants de conception CAD, chacune pondérée par une projection de la distance signée entre l'élément du maillage et le composant de conception CAD respectif.

**3.** Procédé selon la revendication 2, dans lequel la projection est une fonction qui met en correspondance ] - ∞; +∞[ sur [0 ; 1] et qui présente des dérivées du premier ordre bien définies, dans lequel, éventuellement, la fonction est une projection de Heaviside lisse.

4. Procédé selon l'une quelconque des revendications 2 à 3, dans lequel la combinaison pondérée est une somme pondérée.

5. Procédé selon la revendication 4, dans lequel

$$M_{ik} = \frac{\sum_{j \in \Omega_{comp}} H_{ij}\, m_{ijk}}{\sum_{j \in \Omega_{comp}} H_{ij}}$$

$\forall\, i \in \Omega_{mesh}, k \in \Omega_{type}$
où $\Omega_{type}$ est l'ensemble de la ou des propriétés de matériau anisotrope de la pièce composite, $M_{ik}$ est la valeur de propriétés de matériau locales d'une propriété de matériau $k$ dans $\Omega_{type}$ à l'élément $i$ du maillage $\Omega_{mesh}$ du modèle de conception CAD, $j$ est un composant, $\Omega_{comp}$ est l'ensemble de tous les composants, $H_{ij}$ sont des poids, et $m_{ijk}$ sont les propriétés de matériau des composants.

6. Procédé selon la revendication 5, dans lequel chaque poids $H_{ij}$ est défini par la projection de Heaviside lisse suivante donnée par la formule

$$H_{ij} = \frac{1}{1 + e^{\frac{SDF_{ij}}{0.2\alpha \times 2l}}},$$

$\forall\, i \in \Omega_{mesh}, j \in \Omega_{comp}$
où $\alpha \geq 0$ est un coefficient de pente de la projection de Heaviside lisse, où $l$ est une taille moyenne des éléments dans le maillage, et $SDF_{ij}$ est la valeur de distance signée de l'élément $i$ au composant $j$, la valeur de distance signée étant une distance de l'élément $i$ à la surface extérieure du composant $j$, la valeur ayant un signe lorsque l'élément correspond à une partie intérieure du composant et le signe opposé lorsque l'élément correspond à une partie extérieure du composant.

7. Procédé selon la revendication 5 ou 6, dans lequel chaque dérivée approximée respective $\frac{\partial M_{ik}}{\partial CAD_p}$ du ou des champs de propriétés de matériau par rapport à un paramètre de conception CAD est du type :

$$\frac{\partial M_{ik}}{\partial CAD_p} \cong \frac{M_{ik}\left(CAD_p + \frac{h_p}{2}\right) - M_{ik}\left(CAD_p - \frac{h_p}{2}\right)}{h_p},$$

$\forall i \in \Omega_{mesh}, k \in \Omega_{type}, p \in \Omega_{param}$
où $CAD_p$ est le paramètre de conception CAD respectif, où $h_p > 0$ est une petite perturbation, où $\Omega_{param}$ est un ensemble des paramètres de conception CAD.

8. Procédé selon la revendication 7, dans lequel chaque sensibilité $\frac{\partial KPI_n}{\partial CAD_p}$ est du type :

$$\frac{\partial KPI_n}{\partial CAD_p} = \frac{\partial KPI_n}{\partial M_{ik}} \frac{\partial M_{ik}}{\partial CAD_p},$$

$\forall n \in \Omega_{perfo}, p \in \Omega_{param}$
où $\Omega_{perfo}$ est l'ensemble du ou des indicateurs de performance d'utilisation et/ou de fabrication et $KPI_n$ désigne la valeur de l'indicateur de performance d'utilisation et/ou de fabrication $n \in \Omega_{perfo}$.

9. Procédé selon l'une quelconque des revendications 2 à 8, dans lequel le ou les composants de conception CAD sont constitués de plusieurs composants.

**10.** Procédé selon l'une quelconque des revendications 1 à 9, dans lequel la ou les propriétés de matériau anisotrope sont constituées de plusieurs propriétés de matériau anisotrope.

**11.** Procédé selon l'une quelconque des revendications 1 à 10, dans lequel l'au moins un paramètre de conception CAD influençant la ou les propriétés de matériau anisotrope de la pièce composite satisfait une contrainte d'un processus de fabrication pour fabriquer la pièce composite.

**12.** Programme informatique comprenant des instructions qui, lorsqu'elles sont exécutées par un système informatique, amènent le système informatique à mettre en œuvre le procédé selon l'une quelconque des revendications 1 à 11.

**13.** Support de stockage de données lisible par ordinateur sur lequel est enregistré le programme informatique selon la revendication 12.

**14.** Système informatique comprenant un processeur couplé à une mémoire, sur laquelle mémoire est enregistré le programme informatique selon la revendication 12.

Anisotropic wood beams having the fibers aligned along the beam length direction

Isotropic "void" space

Isotropic plastic hubs

## FIG. 1

Isotropic "void" space

Isotropic foam core

Anisotropic conformal composite laminate

## FIG. 2

## FIG. 3

## FIG. 4

Control point of the
CAD B-Spline shape

(a) Initial L-Shape bracket design

FIG. 5

(b) Optimized L-Shape bracket design excluding the derivatives of the anisotropic fibers in the optimization workflow

FIG. 6

(c) Optimized L-Shape bracket design
consistently including the derivatives of the
anisotropic fibers in the optimization workflow

FIG. 7

FIG. 8

Initial design shape showing the isotropic foam core (dark gray) and conformal anisotropic laminated sheets (light gray)

Initial fiber orientations shown for the cross section near the shear web corner

Initial fiber orientations shown for the top view at the trailing edge of the blade

FIG. 9

FIG. 10

FIG. 11

Fiber orientation in the initial design

Fiber orientation in the optimized design

# FIG. 12

Initial frame structure

Isotropic
plastic hubs

Parameterized hub and
range of motion in the
optimization

Anisotropic wood beams having the fibers
aligned along the beam length direction

# FIG. 13

Optimized frame structure

# FIG. 14

Frame structure shown for the (a) initial configuration and for optimization iteration (b) 5 and (c) 25, respectively. The scaled deformations are displayed and colored by the displacement magnitudes.

FIG. 15

## FIG. 16

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2023274048 A1
- US 2023114354 A1
- EP 3327593 A
- EP 21306754

**Non-patent literature cited in the description**

- **BENDSØE MP** ; **SIGMUND O**. Topology optimization theory, methods, and applications. Springer, 2003
- **OLHOFF N** ; **BENDSØE MP** ; **RASMUSSEN J**. On CAD-integrated structural topology and design optimization. *Comput Methods Appl Mech Eng.*, 1991, vol. 89 (1)
- **OLHOFF N** ; **BENDSØE MP** ; **RASMUSSEN J**. On CAD-integrated structural topology and design optimization. *Comput Methods Appl Mech Eng*, 1991, vol. 89 (1)
- **FALLAHPOUR M** ; **HARBRECHT H**. Shape optimization for composite materials in linear elasticity. *Optimization and Engineering*, 2022, vol. 24 (7)
- **OLHOFF N** ; **RASMUSSEN J** ; **LUND E**. A Method of "Exact" Numerical Differentiation for Error Elimination in Finite-Element-Based Semi-Analytical Shape Sensitivity Analyses. *Mech. Struct. & Mach*, 1993, vol. 21 (1)
- **BRUYNEEL M** ; **FLEURY C**. Composite structures optimization using sequential convex programming. *Advances in Engineering Software*, 2002, 33
- **JOHANSEN L** ; **LUND, E**. Optimization of laminated composite structures using delamination criteria and hierarchical models. *Struct Multidisc Optim*, 2009, 38
- **PEDERSEN P**. On thickness and orientational design with orthotropic materials.. *Struct Optim.*, 1991, 3
- **SJØLUND JH** ; **LUND E**. Structural gradient-based optimization of wind turbine blades with fixed outer geometry. *Composite structures*, 2018, 203
- **GHIASI H** ; **PASINI D** ; **LESSARD L**. Optimum stacking sequence design of composite materials Part I: Constant stiffness design. *Composite Structures*, 2009, vol. 90 (1)
- **GHIASI H** ; **FAYAZBAKHSH K** ; **PASINI D** ; **LESSARD L**. *Optimum stacking sequence design of composite materials Part II: Variable stiffness design*, 2010, vol. 93 (1)

- **GANDHI Y** ; **MINAK GA**. Review on Topology Optimization Strategies for Additively Manufactured Continuous Fiber-Reinforced Composite Structures. *Appl. Sci.*, 2022, 12
- **XU Y** ; **ZHU J** ; **WU Z** ; **CAO Y** ; **ZHAO Y** ; **ZHANG W.** A review on the design of laminated composite structures: constant and variable stiffness design and topology optimization. *Adv Compos Hybrid Mater*, 2018, vol. 1 (3), 460
- **HVEJSEL CF** ; **LUND E**. Material interpolation schemes for unified topology and multimaterial optimization. *Struct Multidisc Optim*, 2011, vol. 43 (6)
- **HOZIC D** ; **THORE C** ; **CAMERON C** ; **SAHBI LOUKIL M**. A new method for simultaneous material and topology optimization of composite laminate structures using Hyperbolic Function Parametrization. *Composite structures*, 2021, 276
- **ALLAIRE G** ; **DELGADO G**. Stacking sequence and shape optimization of laminated composite plates via a level-set method. *J Mech Phys Solids*, 2016, 97
- **YANAN X** ; **YUNKAI G** ; **CHI W** ; **JIANGUANG F** ; **GUANG-YONG S** ; **GRANT P S** ; **QING L.** Concurrent optimization of topological configuration and continuous fiber path for composite structures - A unified level set approach. *Computer Methods in Applied Mechanics and Engineering*, 2022, 399
- **SMITH H** ; **NORATO J**. Topology optimization with discrete geometric components made of composite materials. *Computer Methods in Applied Mechanics and Engineering*, 2021, 376
- **SMITH H** ; **NORATO J**. Topology optimization of structures made of fiber-reinforced plates. *Struct Multidisc Optim*, 2022, 65
- **GREIFENSTEIN J** ; **LETOURNEL E** ; **STINGL M** ; **WEIN F**. *Efficient spline design via feature-mapping for continuous fiber-reinforced structures*, 2023, 66